# EUROPEAN PATENT APPLICATION

(11) **EP 3 816 301 A1**
(43) Date of publication of application: **05.05.2021**
(21) Application number: 19206611.6
(22) Date of filing: 31.10.2019
(51) Int. Cl.: C12Q 1/6883

(54) **DETERMINING THE SENSITIVITY OF SKIN TO UV RADIATION**

(71) Applicant: BEIERSDORF AG, 20245 Hamburg (DE)
(72) Inventor: Holzscheck, Nicholas, 22549 Hamburg (DE); Röck, Katharina, 22767 Hamburg (DE); Winnefeld, Marc, 22880 Wedel (DE); Schläger, Torsten, 22395 Hamburg (DE)
(74) Representative: Haseltine Lake Kempner LLP

(57) **Abstract**

The present invention concerns methods for predicting MED of a subject's skin, comprising either determining the methylation levels of at least 2 CpG sites selected from certain genes in a skin sample obtained from the subject, and predicting the subject's MED based on the determined methylation levels using a machine learning model trained on data comprising known MEDs and corresponding known methylation levels of the at least 2 CpG sites, and/or determining the RNA expression levels of at least 2 genes selected from certain genes in a skin sample obtained from the subject, and predicting the subject's MED based on the determined RNA expression levels using a machine learning model trained on data comprising known MEDs and corresponding known RNA expression levels of the at least 2 genes. The invention further relates to computer programs for carrying out the methods of the invention.

## Description

### FIELD OF THE INVENTION

The present invention relates to determining the sensitivity of a subject's skin to UV radiation by analysing gene expression or epigenetic markers. The level of RNA expression of particular genes or the methylation level of particular CpG sites in a skin sample from the subject are analysed to accurately predict the minimal erythema dose (MED) of a subject's skin, which is an indicator of the UV sensitivity of the subject's skin. Methods of the invention can achieve a mean absolute error (MAE) as low as 7.85 mJ/cm² (gene expression) or 4.18 mJ/cm² (methylation).

### BACKGROUND OF THE INVENTION

It is known that exposure of the skin to radiation from the ultraviolet (UV) region of the light spectrum can have harmful effects on the skin, including permanent skin damage, discoloration, and premature aging, as well as DNA damage, which can lead to the development of skin cancer.

The sensitivity/tolerance of the skin to UV irradiation can vary widely between individuals. Stratifying individuals by the sensitivity of skin to UV can be useful for assessing their risk of skin damage and cancer, for determining appropriate UV protection strategies, and for determining appropriate doses of therapeutic UV (e.g. PUVA therapy).

The Fitzpatrick phototyping scale categorises subjects as phototype I-VI based on their skin's complexion and propensity to tanning and burning in response to UV radiation (Fitzpatrick (1975) Soleil et peau, J Med Esthet. (2):33-34; Eilers et al (2013) Accuracy of Self-report in Assessing Fitzpatrick Skin Phototypes I through VI, JAMA Dermatol, 149(11):1289-1294). This is a semi-quantitative and highly subjective way of predicting an individual's skin's sensitivity to UV and is generally only used when a very fast assessment is required. Moreover, the sensitivity of the skin to UV radiation can vary significantly even between people with the same phenotypic skin type.

The current gold-standard way of determining the sensitivity of an individual's skin to UV irradiation is measuring their minimal erythema dose (MED) (Heckman et al (2013) Minimal Erythema Dose (MED) Testing, J Vis Exp. (75): 50175). One MED corresponds to the lowest UV dose (measured in mJ/cm²), which causes erythema (redness) or oedema (swelling) of the skin 24-48 hours after UV exposure. This is typically determined by irradiating several patches of skin with different doses of UV light and assessing 24 hours later which was the lowest dose causing erythema.

This method has several disadvantages: the method requires the skin to be irradiated and burned (risking permanent skin and DNA damage); the precision of the method is low and depends on the doses tested; the method is subjective; the method is slow and requires repeated patient visits to a clinic; the method cannot be performed on skin already exposed to UV radiation.

So far, there is no precise way to determine the tolerance of an individual's skin to UV radiation without irradiating the skin. It is desirable to provide a method for determining an individual's tolerance/sensitivity to UV radiation without prior irradiation.

### SUMMARY OF THE INVENTION

The present invention is defined in the appended claims.

In accordance with a first aspect, there is provided a method for predicting the minimal erythema dose (MED) of a subject's skin comprising:
a)
   i) determining the RNA expression levels of at least two genes selected from the genes in Table 1 in a skin sample obtained from the subject, and
   ii) predicting the subject's MED based on the determined RNA expression levels using a machine learning model trained on data comprising known MEDs and corresponding known RNA expression levels of the at least two genes; and/or
b)
   i) determining the methylation levels of at least two CpG sites selected from the genes in Table 3 in a skin sample obtained from the subject, and
   ii) predicting the subject's MED based on the determined methylation levels using a machine learning model trained on data comprising known MEDs and corresponding known methylation levels of the at least two CpG sites.

In accordance with a second aspect, there is provided a computer program comprising instructions which, when the program is executed by a computer, cause the computer to carry out the following steps:
a)
   i) inputting the RNA expression levels of at least two genes selected from the genes in Table 1 in a skin sample obtained from the subject;
   ii) predicting the subject's MED based on the determined RNA expression levels using a machine learning model trained on data comprising known MEDs and corresponding known RNA expression levels of the at least two genes; and
   iii) outputting the predicted MED, and/or
b)
   i) inputting the methylation levels of at least two CpG sites selected from the genes in Table 3 in a skin sample obtained from the subject;
   ii) predicting the subject's MED based on the determined methylation levels using a machine learning model trained on data comprising known MEDs and corresponding known methylation levels of the at least two CpG sites; and
   iii) outputting the predicted MED.

In a further aspect, there is provided a method for preventing damage to a subject's skin by UV radiation, the method comprising:
A) receiving or obtaining a skin sample from a subject;
B)
   i)
      a) determining the RNA expression levels of at least two genes selected from the genes in Table 1 in a skin sample obtained from the subject; and
      b) predicting the subject's MED based on the determined RNA expression levels using a machine learning model trained on data comprising known MEDs and corresponding known RNA expression levels of the at least two genes, and/or
   ii)
      a) determining the methylation levels of at least two CpG sites selected from the genes in Table 3 in a skin sample obtained from the subject; and
      b) predicting the subject's MED based on the determined methylation levels using a machine learning model trained on data comprising known MEDs and corresponding known methylation levels of the at least two CpG sites, and
C) administering an effective amount of a UV protectant to the subject.

In a further aspect, there is provided a computer-readable medium comprising instructions which, when executed by a computer, cause the computer to carry out the following steps:
a)
   i) inputting the RNA expression levels of at least two genes selected from the genes in Table 1 in a skin sample obtained from the subject;
   ii) predicting the subject's MED based on the determined RNA expression levels using a machine learning model trained on data comprising known MEDs and corresponding known RNA expression levels of the at least two genes, and
   iii) outputting the predicted MED, and/or
b)
   i) inputting the methylation levels of at least two CpG sites selected from the genes in Table 3 in a skin sample obtained from the subject;
   ii) predicting the subject's MED based on the determined methylation levels using a machine learning model trained on data comprising known MEDs and corresponding known methylation levels of the at least two CpG sites; and
   iii) outputting the predicted MED.

Certain aspects and embodiments of the present invention may provide one or more of the following advantages:
- desired objectivity of UV sensitivity determination via MED prediction;
- desired precision of UV sensitivity determination via MED prediction;
- desired reproducibility of UV sensitivity determination via MED prediction;
- desired ability to determine UV sensitivity without harmful exposure of subjects to UV radiation;
- desired ability to determine UV sensitivity without subject being present throughout process.

The details, examples, and preferences provided in relation to any particular one or more of the stated aspects of the present invention apply equally to all aspects of the present invention. Any combination of embodiments, examples, and preferences described herein in all possible variations thereof is encompassed by the present invention unless otherwise indicated herein, or otherwise clearly contradicted by context.

### BRIEF DESCRIPTION OF THE DRAWINGS

The invention will further be illustrated by reference to the following figures:
Figure 1 shows the correlation between MED determined using the known, standardised method (x-axis) and predicted MED based on the RNA expression levels of the 20 genes in Table 2, set A (y-axis). The MAE of this model was 7.85 mJ/cm².
Figure 2 shows the correlation between MED determined using the known, standardised method (x-axis) and predicted MED (y-axis) based on the methylation levels of the 18 CpG sites in Table 4. The MAE of this model was 4.18 mJ/cm².
It is understood that the following description and references to the figures concern exemplary embodiments of the present invention and shall not be limited on the scope of the claims.

### DETAILED DESCRIPTION

The present invention is based on the finding that the RNA expression level of particular genes and/or the methylation level of particular CpG sites can be used to determine the sensitivity of a subject's skin to UV radiation by predicting the MED of a subject's skin.

### Minimal Erythema Dose (MED)

As used herein, "the sensitivity of a subject's skin to UV radiation" refers to how readily a subject's skin (including DNA) is damaged by UV radiation.

The sensitivity of a subject's skin to UV radiation can be represented by a minimal erythema dose (MED). The "minimal erythema dose" is the minimal dose of UV (measured in mJ/cm²) which results in perceptible erythema (redness) and/or oedema (swelling) of the skin 24 to 48 hours after exposure to UV radiation. The known, standardised method for determining MED is provided in ISO 2444:2010.

As used herein, "ultraviolet radiation" ("UV radiation") refers to electromagnetic radiation with a wavelength of from about 100 nm to about 400 nm, including UVC radiation (from about 100 to about 280nm), UVB radiation (from about 280 to about 315nm), and UVA radiation (from about 315nm to about 400nm).

### Subjects and sampling

As used herein, "a subject" refers to a human subject. In certain embodiments, the subject may be at least 16, 18, or 30 years old.

In certain embodiments, the subject may be phototype I-VI on the Fitzpatrick scale (I meaning always burns, never tans; II meaning burns easily, then develops a light tan; III meaning burns moderately, then develops a light tan; IV meaning burns minimally to rarely, then develops a moderate tan; V meaning never burns, always develops a dark tan; VI meaning never burns, no noticeable change in appearance). In certain embodiments, the subject may be phototype I-IV on the Fitzpatrick scale.

In the present invention, it is not necessary for the subject's skin to have been exposed to UV radiation before sampling. Nevertheless, the subject's skin may have previously been exposed to UV radiation and may be tanned or burnt at the time of sampling.

Ideally, the subject had/has not taken anti-histamine or anti-inflammatory drugs within two weeks prior to skin sampling.

As used herein, "a skin sample" refers to a sample comprising skin cells.

In certain embodiments, the skin cells may have been/may be obtained by harvesting the entire skin sample required from the individual. Harvesting a sample from the individual may be carried out using suction blistering, punch biopsy, shave biopsy or during any surgical procedure such as plastic surgery, lifting, grafting, or the like. In certain embodiments, the sample may have been/may be obtained by suction blistering.

In certain embodiments, the skin cells may have been/may be obtained by culturing the skin cells using an *in vitro* method. Skin cells may have been/may be cultured from a small sample of skin cells harvested from an individual. The harvested human skin cells may have been/ may be grown *in vitro* in a vessel such as a petri dish in a medium or substrate that supplies essential nutrients.

The skin samples may have been/may be obtained from the epidermis or dermis. Hence, the skin sample may comprise, consist, or consist essentially of epidermal cells and/or dermal cells. In certain embodiments, the skin sample may comprise, consist, or consist essentially of epidermal cells. The skin cells may comprise a mixture of harvested cells and cultured cells.

### RNA expression level of genes

The RNA expression level of particular genes can be determined, for example, by RNA-Seq (e.g. using Illumina's® TruSeq RNA Library Prep Kit and HiSeq system), RT-qPCR, SAGE, EST sequencing, or hybridisation-based methods such as microarrays.

The RNA expression level of a particular gene can be measured in transcripts per million (TPM). A value of x TPM means that for every 1 million RNA molecules in the sample, x came from the gene of interest.

The inventors of the present invention identified 200 genes whose RNA expression levels individually exhibit a strong linear correlation with MED (see Table 1). The inventors of the present invention also identified sets of 18-20 genes whose RNA expression levels can be used to predict MED with high accuracy (see Table 2).

The inventors surprisingly and advantageously found that the RNA expression level of as few as 2 of the genes in Table 1 could be used to accurately predict MED. MAEs of less than about 25 mJ/cm² were consistently achieved when using from 2 to 200 genes selected from Table 1 to predict MED.

Accordingly, in certain embodiments, the methods of the present invention may comprise determining the RNA expression level of at least 2 genes selected from Table 1 or 2. In certain embodiments, the methods of the present invention may comprise a step of determining the RNA expression level of from 2 to 200, 2 to 150, 2 to 100, 2 to 50, or 2 to 20 genes selected from Table 1 or 2. In certain embodiments, the genes may comprise or consist of: ENSG00000197978 and ENSG00000277060; ENSG00000197978 and ENSG00000100376; ENSG00000197978 and ENSG00000172799; ENSG00000197978 and ENSG00000166670; or ENSG00000172799 and ENSG00000159247. Such methods using at least 2 genes may achieve an absolute error of about 25 mJ/cm² or less, about 20 mJ/cm² or less, about 15 mJ/cm² or less, about 10 mJ/cm² or less, or about 5 mJ/cm² or less.

In certain embodiments, the methods of the present invention may comprise determining the RNA expression level of at least 5 genes selected from Table 1 or 2. In certain embodiments, the methods of the present invention may comprise a step of determining the RNA expression level of from 5 to 200, 5 to 150, 5 to 100, 5 to 50, or 5 to 20 genes selected from Table 1 or 2. In certain embodiments, the genes may comprise or consist of: ENSG00000197978, ENSG00000277060, ENSG00000100376, ENSG00000166670, and ENSG00000172799; ENSG00000197978, ENSG00000159247, ENSG00000100376, ENSG00000166670, and ENSG00000172799; ENSG00000277060, ENSG00000100376, ENSG00000166670, ENSG00000172799, and ENSG00000159247; ENSG00000197978, ENSG00000100376, ENSG00000166670, ENSG00000106392, and ENSG00000159247; or ENSG00000197978, ENSG00000100376, ENSG00000172799, ENSG00000159247, and ENSG00000260075. Such methods using at least 5 genes may achieve an absolute error of about 25 mJ/cm² or less, about 20 mJ/cm² or less, about 15 mJ/cm² or less, about 13 mJ/cm² or less, about 10 mJ/cm² or less, or about 5 mJ/cm² or less.

In certain embodiments, the methods of the present invention may comprise determining the RNA expression level of at least 10 genes selected from Table 1 or 2. In certain embodiments, the methods of the present invention may comprise a step of determining the RNA expression level of from 10 to 200, 10 to 150, 10 to 100, 10 to 50, or 10 to 20 genes selected from Table 1 or 2. In certain embodiments, the genes may comprise or consist of: ENSG00000197978, ENSG00000277060, ENSG00000100376, ENSG00000166670, ENSG00000172799, ENSG00000106392, ENSG00000112139, ENSG00000130779, ENSG00000159247, and ENSG00000260075; ENSG00000197978, ENSG00000277060, ENSG00000166670, ENSG00000172799, ENSG00000106392, ENSG00000159247, ENSG00000100376, ENSG00000260075, ENSG00000188818, and ENSG00000169282; ENSG00000166670, ENSG00000172799, ENSG00000106392, ENSG00000100376, ENSG00000260075, ENSG00000188818, ENSG00000115602, ENSG00000112139, ENSG00000224472, and ENSG00000233913; or ENSG00000277060, ENSG00000166670, ENSG00000106392, ENSG00000159247, ENSG00000100376, ENSG00000260075, ENSG00000169282, ENSG00000126890, ENSG00000176933, and ENSG00000134184. Such methods using at least 10 genes may achieve an absolute error of about 25 mJ/cm² or less, about 20 mJ/cm² or less, about 15 mJ/cm² or less, about 10 mJ/cm² or less, or about 5 mJ/cm² or less.

In certain embodiments, the methods of the present invention may comprise determining the RNA expression level of at least 18 genes selected from Table 1 or 2. In certain embodiments, the methods of the present invention may comprise a step of determining the RNA expression level of from 18 to 200, 18 to 150, 18 to 100, or 18 to 50 genes selected from Table 1 or 2. In certain embodiments, the genes may comprise or consist of 18 genes selected from set A, B, C, D, E, F, G, H, I, J, K, L, M, N, O, P, Q, or R in Table 2. In one example, the genes may comprise of consist of 18 genes selected from set A in Table 2. Such methods using at least 18 genes may achieve an absolute error of about 25 mJ/cm² or less, about 20 mJ/cm² or less, about 15 mJ/cm² or less, about 10 mJ/cm² or less, about 8 mJ/cm² or less, or about 5 mJ/cm² or less.

In certain embodiments, the methods of the present invention may comprise determining the RNA expression level of at least 20 genes selected from Table 1 or2. In certain embodiments, the methods of the present invention may comprise a step of determining the RNA expression level of from 20 to 200, 20 to 150, 20 to 100, or 20 to 50 genes selected from Table 1 or 2. In certain embodiments, the genes may comprise or consist of the genes provided in set A, D, G, H, N, O, P, or R in Table 2. In one example, the genes may comprise of consist of the genes provided in set A in Table 2. Such methods using at least 20 genes may achieve an absolute error of about 25 mJ/cm² or less, about 20 mJ/cm² or less, about 15 mJ/cm² or less, about 10 mJ/cm² or less, about 8 mJ/cm² or less, or about 5 mJ/cm² or less.

**Table 1 - 200 predictive genes:**

| **Ensembl identifier** | **Gene name** |
|---|---|
| ENSG00000009950 | MLXIPL |
| ENSG00000100376 | FAM118A |
| ENSG00000115602 | IL1RL1 |
| ENSG00000134184 | GSTM1 |
| ENSG00000142973 | CYP4B1 |
| ENSG00000156603 | MED19 |
| ENSG00000159247 | NA |
| ENSG00000169282 | KCNAB1 |
| ENSG00000172799 | NA |
| ENSG00000176933 | NA |
| ENSG00000188818 | ZDHHC11 |
| ENSG00000197978 | GOLGA6L9 |
| ENSG00000198744 | NA |
| ENSG00000224472 | TCF19 |
| ENSG00000233913 | NA |
| ENSG00000260075 | NA |
| ENSG00000276822 | NA |
| ENSG00000277263 | HLA-DRA |
| ENSG00000277789 | TMC4 |
| ENSG00000278053 | DDX52 |
| ENSG00000134201 | GSTM5 |
| ENSG00000115590 | IL1R2 |
| ENSG00000232957 | HLA-DOA |
| ENSG00000142178 | SIK1 |
| ENSG00000185829 | ARL17A |
| ENSG00000166670 | MMP10 |
| ENSG00000232333 | NA |
| ENSG00000095203 | EPB41L4B |
| ENSG00000106392 | C1GALT1 |
| ENSG00000214756 | CSKMT |
| ENSG00000180787 | ZFP3 |
| ENSG00000175787 | ZNF169 |
| ENSG00000251553 | NA |
| ENSG00000277060 | NLRP2 |
| ENSG00000228546 | NA |
| ENSG00000112139 | MDGA1 |
| ENSG00000136158 | SPRY2 |
| ENSG00000166483 | WEE1 |
| ENSG00000135913 | USP37 |
| ENSG00000242498 | ARPIN |
| ENSG00000205057 | CLLU1OS |
| ENSG00000164066 | INTU |
| ENSG00000109787 | KLF3 |
| ENSG00000119938 | PPP1R3C |
| ENSG00000276276 | ARL17A |
| ENSG00000282604 | CCL4L2 |
| ENSG00000196862 | RGPD4 |
| ENSG00000276070 | CCL4L2 |
| ENSG00000276125 | CCL4L2 |
| ENSG00000256704 | NA |
| ENSG00000233448 | NA |
| ENSG00000168386 | FILIP1L |
| ENSG00000275313 | CCL4L2 |
| ENSG00000141040 | ZNF287 |
| ENSG00000008438 | PGLYRP1 |
| ENSG00000197728 | RPS26 |
| ENSG00000126890 | CTAG2 |
| ENSG00000179431 | FJX1 |
| ENSG00000249755 | NA |
| ENSG00000230395 | NA |
| ENSG00000281879 | ABO |
| ENSG00000147324 | MFHAS1 |
| ENSG00000257987 | TEX49 |
| ENSG00000130779 | CLIP1 |
| ENSG00000152784 | PRDM8 |
| ENSG00000110031 | LPXN |
| ENSG00000111145 | ELK3 |
| ENSG00000203837 | PNLIPRP3 |
| ENSG00000196581 | AJAP1 |
| ENSG00000147896 | IFNK |
| ENSG00000145882 | PCYOX1L |
| ENSG00000218208 | NA |
| ENSG00000123496 | IL13RA2 |
| ENSG00000238083 | LRRC37A2 |
| ENSG00000144834 | TAGLN3 |
| ENSG00000262961 | NA |
| ENSG00000135211 | TMEM60 |
| ENSG00000260261 | NA |
| ENSG00000175170 | NA |
| ENSG00000214357 | NEURL1B |
| ENSG00000096696 | DSP |
| ENSG00000064309 | CDON |
| ENSG00000128340 | RAC2 |
| ENSG00000196611 | MMP1 |
| ENSG00000270228 | NA |
| ENSG00000073969 | NSF |
| ENSG00000276262 | NSF |
| ENSG00000226124 | FTCDNL1 |
| ENSG00000134363 | FST |
| ENSG00000256043 | CTSO |
| ENSG00000263238 | CTSO |
| ENSG00000127129 | EDN2 |
| ENSG00000228987 | HLA-DRA |
| ENSG00000274471 | NA |
| ENSG00000143067 | ZNF697 |
| ENSG00000129673 | AANAT |
| ENSG00000213225 | NA |
| ENSG00000128578 | STRIP2 |
| ENSG00000132326 | PER2 |
| ENSG00000164764 | SBSPON |
| ENSG00000105509 | HAS1 |
| ENSG00000229391 | HLA-DRB6 |
| ENSG00000185567 | AHNAK2 |
| ENSG00000104427 | ZC2HC1A |
| ENSG00000025772 | TOMM34 |
| ENSG00000134817 | APLNR |
| ENSG00000234224 | TMEM229A |
| ENSG00000025156 | HSF2 |
| ENSG00000071246 | VASH1 |
| ENSG00000278312 | LENG9 |
| ENSG00000112299 | VNN1 |
| ENSG00000188393 | CLEC2A |
| ENSG00000248351 | NA |
| ENSG00000182472 | CAPN12 |
| ENSG00000228887 | NA |
| ENSG00000271415 | NA |
| ENSG00000198535 | C2CD4A |
| ENSG00000176681 | LRRC37A |
| ENSG00000214274 | ANG |
| ENSG00000171310 | CHST11 |
| ENSG00000272916 | NDST2 |
| ENSG00000126217 | MCF2L |
| ENSG00000154914 | USP43 |
| ENSG00000174837 | ADGRE1 |
| ENSG00000233853 | NA |
| ENSG00000081277 | PKP1 |
| ENSG00000135049 | AGTPBP1 |
| ENSG00000198431 | TXNRD1 |
| ENSG00000002587 | HS3ST1 |
| ENSG00000235804 | NA |
| ENSG00000197054 | ZNF763 |
| ENSG00000170260 | ZNF212 |
| ENSG00000225308 | NA |
| ENSG00000151617 | EDNRA |
| ENSG00000164047 | CAMP |
| ENSG00000179912 | R3HDM2 |
| ENSG00000099250 | NRP1 |
| ENSG00000153922 | CHD1 |
| ENSG00000023909 | GCLM |
| ENSG00000162104 | ADCY9 |
| ENSG00000154529 | CNTNAP3B |
| ENSG00000169203 | NA |
| ENSG00000256625 | NA |
| ENSG00000237591 | NA |
| ENSG00000131746 | TNS4 |
| ENSG00000152104 | PTPN14 |
| ENSG00000129048 | ACKR4 |
| ENSG00000149968 | MMP3 |
| ENSG00000123700 | KCNJ2 |
| ENSG00000225345 | NA |
| ENSG00000163735 | CXCL5 |
| ENSG00000235289 | NA |
| ENSG00000124208 | TMEM189- |
| ENSG00000253276 | CCDC71 L |
| ENSG00000224941 | TCF19 |
| ENSG00000146242 | TPBG |
| ENSG00000283085 | TPBG |
| ENSG00000144136 | SLC20A1 |
| ENSG00000169403 | PTAFR |
| ENSG00000249930 | NA |
| ENSG00000158623 | COPG2 |
| ENSG00000101335 | MYL9 |
| ENSG00000155858 | LSM11 |
| ENSG00000228672 | PROB1 |
| ENSG00000227689 | NA |
| ENSG00000273604 | EPOP |
| ENSG00000277303 | EPOP |
| ENSG00000278299 | TBC1D3C |
| ENSG00000124496 | TRERF1 |
| ENSG00000261245 | NA |
| ENSG00000169067 | ACTBL2 |
| ENSG00000186907 | RTN4RL2 |
| ENSG00000157111 | TMEM171 |
| ENSG00000131781 | FMO5 |
| ENSG00000108342 | CSF3 |
| ENSG00000196159 | FAT4 |
| ENSG00000270149 | NA |
| ENSG00000211598 | NA |
| ENSG00000087086 | FTL |
| ENSG00000154059 | IMPACT |
| ENSG00000069275 | NUCKS1 |
| ENSG00000255282 | WTAPP1 |
| ENSG00000133169 | BEX1 |
| ENSG00000162670 | BRINP3 |
| ENSG00000212900 | KRTAP3-2 |
| ENSG00000263296 | KRTAP3-2 |
| ENSG00000173237 | C11orf86 |
| ENSG00000130054 | FAM155B |
| ENSG00000234795 | NA |
| ENSG00000198380 | GFPT1 |
| ENSG00000166016 | ABTB2 |
| ENSG00000231286 | HLA-DQB1 |
| ENSG00000267676 | NA |
| ENSG00000135205 | CCDC146 |
| ENSG00000230076 | NA |
| ENSG00000106133 | NSUN5P2 |
| ENSG00000212747 | RTL8B |
| ENSG00000155876 | RRAGA |
| ENSG00000185985 | SLITRK2 |
| ENSG00000142197 | DOPEY2 |

**Table 2 - sets of predictive genes:**

| **Set** | **No. of genes** | **Ensembl identifiers (further information in Table 1)** |
|---|---|---|
| A | 20 | ENSG00000009950, ENSG00000100376, ENSG00000115602, ENSG00000134184, ENSG00000142973, ENSG00000156603, ENSG00000159247, ENSG00000169282, ENSG00000172799, ENSG00000176933, ENSG00000188818, ENSG00000197978, ENSG00000198744, ENSG00000224472, ENSG00000233913, ENSG00000260075, ENSG00000276822, ENSG00000277263, ENSG00000277789, ENSG00000278053 |
| B | 19 | ENSG00000095203, ENSG00000100376, ENSG00000130779, ENSG00000136158, ENSG00000141040, ENSG00000147324, ENSG00000159247, ENSG00000166670, ENSG00000172799, ENSG00000175787, ENSG00000179431, ENSG00000180787, ENSG00000197978, ENSG00000205057, ENSG00000230395, ENSG00000233448, ENSG00000249755, ENSG00000257987, ENSG00000277060 |
| C | 18 | ENSG00000100376, ENSG00000106392, ENSG00000109787, ENSG00000119938, ENSG00000159247, ENSG00000164066, ENSG00000166670, ENSG00000172799, ENSG00000175787, ENSG00000179431, ENSG00000185829, ENSG00000197728, ENSG00000197978, ENSG00000205057, ENSG00000228546, ENSG00000256704, ENSG00000257987, ENSG00000277060 |
| D | 20 | ENSG00000095203, ENSG00000100376, ENSG00000109787, ENSG00000130779, ENSG00000141040, ENSG00000159247, ENSG00000166483, ENSG00000172799, ENSG00000179431, ENSG00000197728, ENSG00000197978, ENSG00000230395, ENSG00000232957, ENSG00000242498, ENSG00000249755, ENSG00000251553, ENSG00000276070, ENSG00000276125, ENSG00000276276, ENSG00000282604 |
| E | 18 | ENSG00000095203, ENSG00000100376, ENSG00000119938, ENSG00000126890, ENSG00000130779, ENSG00000141040, ENSG00000159247, ENSG00000164066, ENSG00000175787, ENSG00000180787, ENSG00000197978, ENSG00000205057, ENSG00000232333, ENSG00000232957, ENSG00000257987, ENSG00000260075, ENSG00000275313, ENSG00000282604 |
| F | 19 | ENSG00000100376, ENSG00000106392, ENSG00000112139, ENSG00000134201, ENSG00000136158, ENSG00000159247, ENSG00000166670, ENSG00000172799, ENSG00000180787, ENSG00000197728, ENSG00000197978, ENSG00000205057, ENSG00000232333, ENSG00000242498, ENSG00000251553, ENSG00000260075, ENSG00000275313, ENSG00000276070, ENSG00000276276 |
| G | 20 | ENSG00000008438, ENSG00000095203, ENSG00000100376, ENSG00000106392, ENSG00000112139, ENSG00000115602, ENSG00000119938, ENSG00000130779, ENSG00000134201, ENSG00000136158, ENSG00000159247, ENSG00000164066, ENSG00000166670, ENSG00000172799, ENSG00000197978, ENSG00000228546, ENSG00000232957, ENSG00000249755, ENSG00000260075, ENSG00000276070 |
| H | 20 | ENSG00000100376, ENSG00000130779, ENSG00000136158, ENSG00000141040, ENSG00000159247, ENSG00000172799, ENSG00000180787, ENSG00000196862, ENSG00000197728, ENSG00000197978, ENSG00000214756, ENSG00000232333, ENSG00000242498, ENSG00000251553, ENSG00000256704, ENSG00000260075, ENSG00000276070, ENSG00000277060, ENSG00000281879, ENSG00000282604 |
| I | 18 | ENSG00000100376, ENSG00000106392, ENSG00000142178, ENSG00000159247, ENSG00000164066, ENSG00000185829, ENSG00000196862, ENSG00000197728, ENSG00000197978, ENSG00000230395, ENSG00000232957, ENSG00000233448, ENSG00000242498, ENSG00000249755, ENSG00000251553, ENSG00000260075, ENSG00000276070, ENSG00000276276 |
| J | 19 | ENSG00000100376, ENSG00000115590, ENSG00000130779, ENSG00000134201, ENSG00000135913, ENSG00000159247, ENSG00000166670, ENSG00000175787, ENSG00000180787, ENSG00000185829, ENSG00000197728, ENSG00000197978, ENSG00000205057, ENSG00000214756, ENSG00000232333, ENSG00000233448, ENSG00000249755, ENSG00000256704, ENSG00000276070 |
| K | 19 | ENSG00000008438, ENSG00000095203, ENSG00000100376, ENSG00000112139, ENSG00000130779, ENSG00000142178, ENSG00000159247, ENSG00000166670, ENSG00000175787, ENSG00000180787, ENSG00000196862, ENSG00000197728, ENSG00000197978, ENSG00000228546, ENSG00000232333, ENSG00000242498, ENSG00000249755, ENSG00000275313, ENSG00000276276 |
| L | 19 | ENSG00000100376, ENSG00000115590, ENSG00000119938, ENSG00000126890, ENSG00000142178, ENSG00000159247, ENSG00000175787, ENSG00000180787, ENSG00000197728, ENSG00000197978, ENSG00000205057, ENSG00000228546, ENSG00000232957, ENSG00000233448, ENSG00000242498, ENSG00000251553, ENSG00000276070, ENSG00000276125, ENSG00000276276 |
| M | 19 | ENSG00000100376, ENSG00000106392, ENSG00000115590, ENSG00000130779, ENSG00000142178, ENSG00000166483, ENSG00000166670, ENSG00000172799, ENSG00000180787, ENSG00000185829, ENSG00000197728, ENSG00000197978, ENSG00000205057, ENSG00000230395, ENSG00000233448, ENSG00000249755, ENSG00000251553, ENSG00000260075, ENSG00000275313 |
| N | 20 | ENSG00000008438, ENSG00000100376, ENSG00000119938, ENSG00000126890, ENSG00000134201, ENSG00000141040, ENSG00000166670, ENSG00000168386, ENSG00000172799, ENSG00000175787, ENSG00000180787, ENSG00000185829, ENSG00000197978, ENSG00000242498, ENSG00000256704, ENSG00000257987, ENSG00000260075, ENSG00000275313, ENSG00000276276, ENSG00000281879 |
| O | 20 | ENSG00000095203, ENSG00000100376, ENSG00000106392, ENSG00000135913, ENSG00000164066, ENSG00000166670, ENSG00000168386, ENSG00000172799, ENSG00000180787, ENSG00000197978, ENSG00000205057, ENSG00000228546, ENSG00000230395, ENSG00000232333, ENSG00000251553, ENSG00000256704, ENSG00000260075, ENSG00000276276, ENSG00000277060, ENSG00000281879 |
| P | 20 | ENSG00000100376, ENSG00000134201, ENSG00000141040, ENSG00000142178, ENSG00000159247, ENSG00000164066, ENSG00000166670, ENSG00000168386, ENSG00000172799, ENSG00000175787, ENSG00000179431, ENSG00000197978, ENSG00000232957, ENSG00000233448, ENSG00000242498, ENSG00000249755, ENSG00000275313, ENSG00000277060, ENSG00000281879, ENSG00000282604 |
| Q | 19 | ENSG00000100376, ENSG00000109787, ENSG00000119938, ENSG00000126890, ENSG00000147324, ENSG00000159247, ENSG00000164066, ENSG00000168386, ENSG00000172799, ENSG00000175787, ENSG00000196862, ENSG00000197978, ENSG00000214756, ENSG00000230395, ENSG00000232333, ENSG00000232957, ENSG00000233448, ENSG00000242498, ENSG00000277060 |
| R | 20 | ENSG00000095203, ENSG00000106392, ENSG00000115602, ENSG00000126890, ENSG00000134201, ENSG00000141040, ENSG00000159247, ENSG00000166670, ENSG00000185829, ENSG00000197728, ENSG00000197978, ENSG00000205057, ENSG00000228546, ENSG00000232957, ENSG00000251553, ENSG00000257987, ENSG00000276070, ENSG00000277060, ENSG00000281879, ENSG00000282604 |

### Methylation level of CpG sites

As used herein, a "CpG site" (also referred to as a CpG dinucleotide) is a cytosine nucleotide immediately followed by a guanine nucleotide in the 5' to 3' direction within a DNA molecule. CpG sites may be in coding or non-coding regions of the genome. CpG sites may be in CpG islands, which are regions having a high density of CpG sites.

The cytosine in a CpG site can be methylated by DNA methyltransferases to become 5-methylcytosine. It is known that methylation of CpG sites within a gene can influence the transcriptional regulation and thus expression of the gene (epigenetic regulation).

The methylation level of particular CpG sites can be determined, for example, by methylation specific PCR, sequence analysis of bisulfite treated DNA, CHIP-sequencing (Illumina Methylation BeadChip Technology), molecular inversion probe assay, Methyl-CAP-sequencing, Next-Generation-sequencing, COBRA-Assay, methylation specific restriction patterns, or MassARRAY assay.

The methylation level of a particular gene can be represented by its M-value, which is the log2 ratio of the intensities of methylated probe versus unmethylated probe. Hence, positive M-values mean that more molecules are methylated than unmethylated, while negative M-values mean the opposite.

The inventors of the present invention identified 200 specific CpG sites whose methylation levels individually exhibit a strong linear correlation with MED (see Table 3). The inventors of the present invention also identified sets of 18-20 CpG sites whose methylation levels can be used to predict MED with high accuracy (see Table 4).

The inventors surprisingly and advantageously found that the methylation level of as few as 2 of the CpG sites in Table 3 could be used to predict MED accurately. MAEs of less than 21 mJ/cm² were consistently achieved when MED was predicted using from 2 to 200 CpG sites selected from Table 3.

Accordingly, in certain embodiments, the methods of the present invention may comprise determining the methylation level of at least 2 CpG sites selected from Table 3 or 4. In certain embodiments, the methods of the present invention may comprise a step of determining the methylation level of from 2 to 200, 2 to 150, 2 to 100, 2 to 50, or 2 to 18 CpG sites selected from Table 3 or 4. In certain embodiments, the CpG sites may comprise or consist of: cg06376130 and cg03953789; cg18269134 and cg00613587; cg06376130 and cg01199135; cg03953789 and cg00492074; or cg06376130 cg18269134 (described further in Table 3). Such methods using at least 2 CpG sites may achieve an absolute error of about 20 mJ/cm² or less, about 15 mJ/cm² or less, about 10 mJ/cm² or less, or about 5 mJ/cm² or less.

In certain embodiments, the methods of the present invention comprise determining the methylation level of at least 5 CpG sites selected from Table 3 or 4. In certain embodiments, the methods of the present invention comprise a step of determining the methylation level of from 5 to 200, 5, to 150, 5 to 100, 5 to 50, or 5 to 18 CpG sites selected from Table 3 or 4. In certain embodiments, the CpG sites may comprise or consist of: cg06376130, cg03953789, cg18269134, cg00613587, and cg01199135; cg06376130, cg18269134, cg00613587, cg01199135, and cg00492074; cg03953789, cg00613587, cg01199135, cg00492074, and cg20271602; cg03953789, cg18269134, cg01199135, cg20707157, and cg22235661; or cg06376130, cg03953789, cg01199135, cg00492074, and cg10094916 (described further in Table 3). Such methods using at least 5 CpG sites may achieve an absolute error of about 20 mJ/cm² or less, about 15 mJ/cm² or less, about 12 mJ/cm² or less, about 10 mJ/cm² or less, or about 5 mJ/cm² or less.

In certain embodiments, the methods of the present invention may comprise determining the methylation level of at least 10 CpG sites selected from Table 3 or 4. In certain embodiments, the methods of the present invention may comprise a step of determining the methylation level of from 10 to 200, 10 to 150, 10 to 100, 10 to 50, or 10 to 18 CpG sites selected from Table 3 or 4. In certain embodiments, the CpG sites may comprise or consist of: cg06376130, cg03953789, cg00613587, cg20707157, cg09218398, cg26096304, cg09174638, cg00492074, cg20271602, and cg22235661; cg03953789, cg18269134, cg00613587, cg01199135, cg20707157, cg00492074, cg20271602, cg22235661, cg24688871, and cg10094916; cg06376130, cg03953789, cg01199135, cg20707157, cg09218398, cg00492074, cg20271602, cg22235661, cg17972013, and cg15224600; or cg18269134, cg00613587, cg01199135, cg20707157, cg00492074, cg20271602, cg22235661, cg24688871, and cg10094916 (described further in Table 3). Such methods using at least 10 CpG sites may achieve an absolute error of about 20 mJ/cm² or less, about 15 mJ/cm² or less, about 10 mJ/cm² or less, about 7 mJ/cm² or less, or about 5 mJ/cm² or less.

In certain embodiments, the methods of the present invention may comprise determining the methylation level of at least 18 CpG sites selected from Table 3 or 4. In certain embodiments, the methods of the present invention may comprise a step of determining the methylation level of from 18 to 200, 18 to 150, 18 to 100, or 18 to 50 CpG sites selected from Table 3 or 4. In certain embodiments, the CpG sites may be selected from the CpG sites provided in set A, B, C, D, E, F, G, H, I, J, K, L, M, N, O, P, Q, or R in Table 4. In one example, the CpG sites may comprise of consist of the 18 CpG sites provided in set A in Table 4. Such methods using at least 18 CpG sites may achieve an absolute error of about 20 mJ/cm² or less, about 15 mJ/cm² or less, about 10 mJ/cm² or less, or about 5 mJ/cm² or less.w

In certain embodiments, the methods of the present invention may comprise determining the methylation level of at least 20 CpG sites selected from Table 3 or 4. In certain embodiments, the methods of the present invention may comprise a step of determining the methylation level of from 20 to 200, 20 to 150, 20 to 100, or 20 to 50 CpG sites selected from Table 3 or 4. In certain embodiments, the CpG sites may comprise or consist of the CpG sites provided in set B, C, D, E, G, H, I, J, K, N, O, P, or R in Table 4. Such methods using at least 18 CpG sites may achieve an absolute error of about 20 mJ/cm² or less, about 15 mJ/cm² or less, about 10 mJ/cm² or less, or about 5 mJ/cm² or less.

**Table 3 - 200 predictive CpG sites:**

| **cgID** | **Associated gene** | **Chrom osome** | **DNA sequence, with CpG site in square brackets at position 61-62** | **SEQ ID** |
|---|---|---|---|---|
| cg24688871 | FAM213B | chr1 | TGGGCATCTCTGCGGAGGTCTGTGCCAGCGACC CGCCTCAGGTGAGCTGGGCCTTGGGGG[CG]CTG CCTGCCAGCCCACGCCCTGCCCCTAGGTCCCCT GGGAGGAGCACTCGGCTTGGCTGG | 1 |
| cg18379824 | CASP9 | chr1 | CCTCTGCGCCTCGCCCCGCCCCCAGGGCCAAGC CTCCCATCACCGCGCCGCCCCAGAACA[CG]CTC CGCGTCACCGCCCCGCCCCAGAACCCGCCCCAC GTCACCGCCCCGCTCCAGAATCCA | 2 |
| cg10094916 | GSTM4 | chr1 | CCTTTTTTGTTTATAGTGTGTGCATGTTTCTGCCA ATGAATCACAGTCTATGAATCTACC[CG]TCCTTGA ATCTCCTGTATAAAACTCTCTGTTTACCCATTCTC TACCTGTGGCTTAGTCAA | 3 |
| cg22165175 | KCNA2 | chr1 | CTCCGGGATCCCTGGGCCGCAGGCCTCCCTCTG AGCCGGGTGGCATTTGAGCTGGGCAGG[CG]CTC | 4 |
| | | | GACCACTGATTTCTCCCCCAAGAGAAAATCCCTC ACATCTCCTCGGCAGGGAGCACA | |
| cg22277636 | DIRC3 | chr2 | TAGGCACCTGGACACATATGCGTGTGCACGCAC GCAAGGCCGCGCACGCTGGGCTCTGGC[CG]GCA CGGCCCTCACGTGCCCTCACGCACGTATGCCCC GCATGGGTTCCCCCAGCGAGCTAT | 5 |
| cg15834295 | ATP2B2 | chr3 | TTCCAAGGTCGCAGGAACCTGGCCTGAAATGGC ATAGCTCCTGAGGCTTGCGGGCTGTC[CG]CCC TCGGATTCGGGTTATTTGAGAGCTGAGCAGCCA AAACACACCGCTCTTCTCTTGTT | 6 |
| cg02754929 | RGS12 | chr4 | AAGGGGCCCAGAGCCATGGAGTGTAGACAGTGG AGGGTGAACACAGAGGGGGCCCAGAGC[CG]TGG AGTGTGGACAGAGAGGACCACCTGGCCTCTCAC CTGCTTGGTCACTTGCTGGGAATG | 7 |
| cg06223984 | UGT2B15 | chr4 | CAAGTGCCAGTTCCTTCCCGGTGTTCAGCCACTG TGTTAATCCTCCACGGGGGCCTGCTA[CG]TGTTG ATCTGGTGAGGTGTTCCACTGGGGCAATTTCCTA CCCAGGAGCGCTCTTTGGATC | 8 |
| cg17972013 | SEMA5A | chr5 | CCAGCGCCCTTCCAGCCAGTGGTGGGGTATGGA TGGGCCTCGCTGGTCCTGGGCATGGTC[CG]GTG CGTCCATAAGGGTTCCATTGAGCTCATCCATAAA ACAAAGAAGCTATTGCAGGCTGC | 9 |
| cg03980424 | NA | chr10 | CCATGCTCCATGGGAATGCTTGACGACTCCAGAT ATCCTATAATTAACTGCAGGGCTTGA[CG]GGGGA CAGAGAAACTCCACCGCATGTTTATTTTTACAAAG AGGCAGCCGCACAGAGGTTG | 10 |
| cg18888319 | NA | chr12 | ACTTCCCTTTGCAGTAAACACATGACCCTGGTAAT CAGTCACTCTCCAACTTGGCTGGTC[CG]GTGTGG TAGAACACACCCTGGGTGACTGTGCAGTTTGGTT GGGAAAATATTTGCCAAGCA | 11 |
| cg04438098 | NA | chr13 | GACAGAGCTCTTACTTCTTCTCTGATTGCTTTAAT GGAATTGACTTAGTTTCCTAACCA[CG]TGATCAC GTTGGGAACCAAGTTCTTACCTTGGCATTTCCCA CAATGCTGGGCACGTGCTC | 12 |
| cg15637874 | HAGHL | chr16 | CACAGCTGGGGCTGCTGCCTGCCAACCTCCCGA GCAGGGCCTCTGTCCCTCACACATGAG[CG]TTTG CATTTCCAGGTGGGAGACAAGTCACAGACACAGA GGAACAGCTCCTGTGTCACCAG | 13 |
| cg06757951 | WWOX | chr16 | AAAACTCATGCCAGTGGTAAAGTTAAGCTGCTTT CTGGTAAGAATTTCAGAATTCCAGGA[CG]TCTCAA ATTTCTGGCTGGTACACCCTGGGCAAGGTCCACA GGCTGGATGGGTTTTGGAAC | 14 |
| cg26881363 | PRPSAP2 | chr17 | TAAAATAGTGCTGGGCCGATTATCCTTTAACAAAT TGGTTGCTTCAACATGTTATGACTG[CG]ATATGTT AAATAGCCATTTGTTTGGACTGCAGTAATTTGGAA TTTGACAGTCAGCTTGTA | 15 |
| cg13957186 | RHBDF2 | chr17 | CAGAGGGAGACTCTGCCTCAAAAACAAACAAAA CCACATATTTTGGGCAGGGTGCAGG[CG]CTCAC ACCTGTAATCCCAGCACTTTGGGAGGCCAAGGTG GGGGAATCATGAGGTCAAGAG | 16 |
| cg22175856 | SLC1A6 | chr19 | AAGTAAATAAAAATATAACTAAAAACAAAATGGCA GGGGAGGGGGAAGGTAAGTCTGTTG[CG]ATGTG | 17 |
| | | | CCCGGTTCTAAGGACAGGTCTTGGCAGGAAAAC GTCAAGTCTCCAGAATCATTCC | |
| cg15224600 | NA | chr20 | AGGGACAGGCCACTGGCATCATTTGTGCTGGAAT GTAAATGACTGACTGGCTCCCTGACG[CG]TGGCT GCTTTCTAAGAGGAGGGCACTCCTGTCTGTGGCT CAGCATGTACTTGCCTCTCTT | 18 |
| cg20464719 | FCHO1 | chr19 | CCCTTTCTCCTCCTCGTCGCCCGAAAACGTGGAG GATTCCGGCCTGGACTCTCCGTCCCA[CG]CGGC ACCTGGCCCCTCCCCAGATTCCTGGGTCCCCCG CCCAGGCACCCCGCAGAGCCCGC | 19 |
| cg15214456 | AP001619.2 | chr21 | ATAACCGTCCCGCCTGCTTCCAAAATGGAACATT TAGCTTGTTCTTTGCTGGCTACTTTG[CG]GCAAAA AGTATGAATCACAGATAGAAGAAAAACAAACTATA AAAAAAATGGTGTTTTTGC | 20 |
| cg09174638 | MSANTD3 | chr9 | GAATAATCAGTTCCCTAATATTAAAGAAAAATCAA TTAACAAATCAGAGTCTAGGCCGGG[CG]TGGTGG CTCACGCCTGTAATCCCAGCACTTTGGGAGGCC GAGGCAGGCAGATCACTTGAG | 21 |
| cg22810163 | CHD7 | chr8 | GGTTGAGTCGTGGTGGTGCGGACGCGCTCGTGC TCGGGAACTATCGGATTAAACTTGAAT[CG]AGTG AAATTACACAAAGGAGCGCCGCGGAGGAGGCGG CCCGGGGACCCGGACACCCTGAA | 22 |
| cg19712277 | MMEL1 | chr1 | AGCTGCCGCTCCAGCTCCCACTCGAGTCCTGGG GAGACAGTGCCCCGCACTGGAGACTGG[CG]GGC CCTGGGGCCCCAGCCCGGCCCCTTGTCGGTGAA TCTGGCCTCCGGAGCCAGGAACTA | 23 |
| cg17379497 | NOC4L | chr12 | GCTATCTTACCCTCCCCCTGGCAGCATTTGAGAG TTCCATACTCCACATCCTCGCCAACA[CG]TCTGTC TATAATTATAGCCGAGCTGCAGACTTTTAAATGTT GGGGAGATACCACGTGAAA | 24 |
| cg19571408 | ADARB1 | chr21 | ACTGGCCAAGCGGGTTTGCTAATCCTTTATCCCA AATTAGACAAACTTCCCACCTAGTAA[CG]AGCATG GCTGGGGGCAAGCCCCTTCCACGTGGCAGGAGA AGAAACCCTAGCACTTTGGTG | 25 |
| cg06376130 | GRTP1 | chr13 | GGCGAGCCAGGCCTGGGAGAATCCGGGGCAGC TCTGGTTCCGAACGGCTTCTTCTCGGTC[CG]CAT CCAATGACATTGTCCTGAGATTTCAGCTGCTTTTC GTGTCCGGGAGGCCCCGGAAAT | 26 |
| cg03518863 | NA | chr16 | GGAACAAGAATACAGGTCCTCTAATGGACAAAGC TCATTCAGGGACCCTCCAATGTGAAT[CG]ACCAG ACACAGTCCTCATGTGTCATGGGAGGGGACAG GCAGCAGCTCCTGGAGCGGAAG | 27 |
| cg07671805 | ANKDD1B | chr5 | GTTGCCGGACACGAGCTCCGTGAGTCCCGGGAC GAGGTCTCAGAAAATCAGGCTGCGGGG[CG]GGC TGGGTCGAAGGGACTTGAGAAGGTACCCAGAGC AGCACCTCCGGACGCTGCAGGAGG | 28 |
| cg01312475 | FHIT | chr3 | AATACATATTTGTTGCATGAATCAAGTGCAGAGGA GGGAAGTAATTTTCCTAAGGCTCCA[CG]GCCAGA AAGTGGCCAGGACTTGAATGTGGTTCTATCTGCT TGCCAAACTCAAATTCTTAA | 29 |
| cg15562220 | SCGN | chr6 | CCTGAATTCCCCAAAGCGCAGAGACAGACAGGAT CTGCCAGGACAGCGCGCAGGGCGGGG[CG]GGG | 30 |
| | | | ACAGGCGCGCCAGGAGCGGGGCGGGCTTCCAG CCGCTGGTTTTGCTGAGGGCTGAGG | |
| cg00613587 | RNF10 | chr12 | TTGTGATTACAGCATGAATCAGCAGCAGAAACTT GCTGACTGGTATTCTGACCTCTAGGC[CG]AGCCA CCTGTTAGAACTAGAGCTAGAAATGAAGATGCCA AGTAATTTAACTGTGAAAAAC | 31 |
| cg16741230 | CATIP-AS2 | chr2 | CTGTCCCCTGCTCCCTTGTTCCCCAGTCCCCTGT GTCATCAAGATGTGGTCAGCGTGGA[CG]GCTTC AGATCCGGGCCCTGGCTACACCTGGCCACACAC AAGGCCATCTGGTCTACCTACT | 32 |
| cg15985703 | GUSBP1 | chr5 | GCACAAATCTCATCCCAAGGGCATAGTTGGTTGC CTGCCAAAAATCAATTTCCTAGTCCA[CG]TTCCTT CACAGACCCTGGTGGTGTTCAGGCATCCTCCTCT CCTCCTTGTAACCTTGTCAT | 33 |
| cg26096304 | CNOT6 | chr5 | TTACTTCATATTCTGCTAAGTCTCTTATAACCCCT CACTGTGATTCCTATCCCCAGCTCC[CG]CTAACC CCCAGCATTTCTGCAAGTGTGAAGTAGACTGGAT TCCTGAAGGTTTTTTTTCTC | 34 |
| cg06509814 | PTK2 | chr8 | CAGACTTGTTTATCGGAGAGCAAACAGGGCTAAC ACAGAAAGCCCTTGTAAAAACAGAA[CG]ACATG AAACAACTATGTATATGCTTCAAATTAACCAATCT ACCAACCGCAAAGCTCTTCC | 35 |
| cg16319140 | NA | chr4 | GAATCTGGCTGTCCCCACAGCTACTTTGCCTGGG GCCCAACTAGGGCAGAGATACAAATA[CG]CCAGG CCATGGCCCCATAAAGTCTGGCCTCAAGGCTGCA GCCCATTTGGAAAACGTTTTA | 36 |
| cg08101922 | NA | chr7 | TTGAGTATTATGCTGATTACCTGCATGACAAAATT ATCTGCACACCAAACCCCTAAAGCA[CG]CAATTTA CCCATGTAACAAAACTGCACATGGACCCCTTGAA CCTAAAATAAAAGTTGGAA | 37 |
| cg09680131 | COL4A2 | chr13 | CACACTGCAAAGGCGGGTGCTTTCAAAATTCACT TTTTCCCACAAGCGGATTCAGAAATG[CG]GAGGT TGCCTGCCGCCTTTTCTACCGGACAACATTCCCG AGTTATTGGTGAGCTGGAATG | 38 |
| cg22235661 | RNA5SP48 4 | chr20 | AGGTGTGTTCAGGGCGGTATGGCTGTAGGCTTCT CATACATCTCACGTGCCACCTCAAGA[CG]TTTGC ACATGCTGTTACCTCTATCTAGAATACTCCCCTCT CCCCTATTCCCCACACTAGA | 39 |
| cg02186126 | CCDC129 | chr7 | ATTCCTCTTGGGGACAAGTCAAAGAATTAGCAAG AACTAGGTTCGAAATGGTCCTTGGGG[CG]GACAG ACTAACTAGAGAAGTCTGCAGGCAGTCACATGCC ATAACTTGTGCCCTGTCTATC | 40 |
| cg00283857 | ZDHHC14 | chr6 | CTGCACACGTGGAACGCAGCATTTCGGGTTGTTT ATTTATCCAGGCGGGCTTTGAGGTTA[CG]GAATA TTTTTAAACAACCAACAGCTGGGGCCAGGGAGCC ACCTCTTCTCTCTCGGATCCT | 41 |
| cg08069338 | SNORA29 | chr6 | CTATACCAGCATGCTGGATGATCTATGAAGAGTT TATACTGTGTATCACATTTACTGACT[CG]GTAAGA TCACACTGTCATATCATGATGTATTTATTAATTTCT CACACTCCCATTTTGAAT | 42 |
| cg00657415 | NA | chr10 | TGTGCCATTTTCCAATCACTCACTCCTCATTCATT CCTCAACTCACCCAGCCTCTACTTT[CG]GCCAGG | 43 |
| | | | TCATTCCGACATTTGCCTCGCTGTGTAATCTGATT GTGGATTGACAACTTATTG | |
| cg22402224 | ORAI2 | chr7 | AGGGATCTCTGAGCAGGGCCTCAGAGATTGATTG GGGTTGGATCAGTTTAGCCCAGTTAG[CG]ATGCA GACGTTCCTGGAATCTGGCCTCCAGGGTGACAAA GGAGAGCTTTTGGCCTGGCTC | 44 |
| cg00698263 | NA | chr17 | CCGGGAACACCGAATCGGAACACTAGAGGGGGC TGAAGTCCCTGATATGGGGGCCTTGAT[CG]CTCT GCCGGGTCTGCAGCTGCAGCCACAGCTAAGCGC TCCCTCGCGCGACGGCTGAAGCT | 45 |
| cg03953789 | NA | chr17 | GGCCTTGATCGCTCTGCCGGGTCTGCAGCTGCA GCCACAGCTAAGCGCTCCCTCGCGCGA[CG]GCT GAAGCTCAGACCCAGAGGCCCGCAGGTCACGAA CCCAAGACCCCAGACCCTCCATGG | 46 |
| cg01873983 | TUFT1 | chr1 | ATCTCTCCACCCCCTCTTGTTTCTATTTTCTCATG GAACGCTAGAAATCTTTTCTTCTTT[CG]CTTCGTT CCTTCTTCCTCTTCTCCTTTCTTCATTCCTTCCTTT TTCTCCCTCCCTTTCTT | 47 |
| cg09218398 | FGFRL1 | chr4 | CGGAGTTCAGAGGAGCCGCCGGGATGCGTGGC CTTTCTGTTCTCTTCTGCGCCTGGGCC[CG]GGC GCTGGGCTTGGACAGGTGTCCAGAGAGACACGC CCTCCATACCTAGGGACCCGGCAC | 48 |
| cg01199135 | SDK2 | chr17 | CTGATAAACTCATGAAAATATGCTCAACTTCCGTC ATAGTGAAAGAAATTCAAATCAAGA[CG]ACAAGAC CATGTTTGCCTAGCAGATCATCAAAGATTTCAGG TGTGGTCAAACCCAGTGT | 49 |
| cg13668823 | BLCAP | chr20 | GCGGGACTGGAGGGCACAGTTGAGGAAGGAGG GTGGTTAAGAAATACAGTGGGGCCCTCT[CG]CTG TCCCTTGCCCAGGGCACTTGCATTCCAGCCTCGC TGCATTTGCTCTCTCGATTCCCC | 50 |
| cg00100025 | SSH2 | chr17 | AGCTCACTCCATTGTAAATATTATAAGCCCTCTGT GTCATGGGAATAACCCTGCCTACGA[CG]TGATGC TGATGTAATAATAATATCTAAAGCTAAGCACCTGG AGGAAGAAGAGAAAGAAGT | 51 |
| cg12642525 | LTBP1 | chr2 | GCTGGATCCTCCTTCCCCAGGACTCTGCACAGTG CAGCAAACACTCTTGGAATGAGGGTA[CG]TTGTA GGCACTATAGGTACCAGCGATTTTAATACGTGAA AATTAAATCTTATAAAACCTT | 52 |
| cg10419493 | NRP1 | chr10 | TGCATTTCCTCTGCTACATATGATGCTTCCAGTAG TAGATACTTGGCCTGCTAACACCAC[CG]TCCCTTT GCAAAACTGTGGAAATACACAGAGGTATTGTTCA TTTCTCCATACTTTGGGAA | 53 |
| cg15452389 | NA | chr6 | AGCCAGGGGCCTCCAGCCAGTGCAGTGTGACCC AGGCGCGCCCTCAGAGCCTGGCGTGGC[CG]GAC TCTGGCTTCTCGGTTGCCCCTGGGTTGTCCCCG GACTGACATCTCTGGGTCTCTTCC | 54 |
| cg10499189 | KIAA0586 | chr14 | CTCTTCATGCAATTGTGATGAAATAATTGACAACT CTTTATGATTCAATTGGGTATGAGA[CG]GTAAGCA AGTAGAGAAGTCAAGATGACTTCAACATTTTGAG ACTGAGAGAAAAATGTTAT | 55 |
| cg06771789 | ISG15 | chr1 | GGCTCCTCCCCATGGGATCTGGAGGGGAGGGGC TGCCTGTGTAATCAGCCCAGCCTCGGA[CG]TCAA | 56 |
| | | | CAATCCATGTTTCTTAGGAAAGGCGGCCAACCCA GGGTCTCCAGTGACACGTTTCC | |
| cg22777490 | PTK2 | chr8 | TGTTTAAAACTACAGTTGTCTACTGGGCTTTCTCC AAATAAGACTGGTATCTATAAAACA[CG]GTATCTG ATTTTATTAACAATTAGACTTGACAGCATAATCTG AACGGAACCTTTAGAAAA | 57 |
| cg20707157 | MEGF6 | chr1 | GAGAGGCAGCCCTCCTCGTCGGGCTGCTGCATC CACCCTCGGCAGCACCTGAGCACGGTC[CG]GGC CTCCGTGGTATACACCTGCCTGTAGCCCATGTAG TAGACGGTTCTAGAAAGAAAGAG | 58 |
| cg18549952 | JADE1 | chr4 | TGACCTACCATTGATTGTGTCCTGAATGAGAAGA GTCTAGGACAACTCTTAGATTTCTGA[CG]TCAAGT AGAAATGGTGGTAATAGTCATCAAAGTAGGAATG TAAGAGGCAGAACAGGTTTG | 59 |
| cg05317498 | UNKL | chr16 | CTGGCGTGGACGTGGCGCAGCTGGCAGGTCCG GGCCTCTGAACTTCACCCCGAATCTGGC[CG]CGC TCAGCAGCCACACTGTGTGGAGACCGTGCTCCA CCACCCAGAGCGGCAGGCGGGAGA | 60 |
| cg08211704 | NA | chr8 | GGGACCAGAGAGCAAGAGAAGGTGGAGAGACTG CGGGCAGAAGCAGCTCCCATTCCTGCA[CG]CACT CTGTGAGGAACACAGGGCCAGGGGCAGAGTCCA CAGAGTCCCAGAAACAGCCTCTC | 61 |
| cg20018782 | MMP14 | chr14 | CCTGGGGCTCTGCTGCTAAGACAGAAGGAGACG AGGGAGAAAGAGAAAGAGGTGTCGCTG[CG]GGG CCTGCTAGGAGCAGCCCGTCTCTGGCCAAACAG AGATCTCATCCCAATTGCCCGGAA | 62 |
| cg01008088 | GPR133 | chr12 | CAGAGACCACTTCATTTGCAGCTTCCTCTCCTCC CGAGCTAAATGGCGCGCGACGAGCCC[CG]CCCT GCCCTCCAGGACATTCATGGCTCGTGCCAGGCA GTGCCCTGGGTCTCCTGCATGCC | 63 |
| cg00492074 | RP11-293I14.2 | chr12 | GTAATTGAATCATGGGACAGGTCTTTCCCGTGCT GTTCTCGTGATAGTGAATAAGTCTCA[CG]AGAGC TGATGGTTTTGTAAAGGGGAGTTCACCTACACAA GCCCCCTTGCCTGCCGCTATG | 64 |
| cg20271602 | FOXN1 | chr17 | TGTATTCCTGGAAATACCTTGGAAGGTTAACATGT CCTTCCATAGCCTGACCTCCATAAG[CG]GCGTGA CCCTGGACAAGTCTTAACGTCTCTGAGCCTCATC TGAAAAGTAAGGTCAACATT | 65 |
| cg18269134 | RP11-47J17.3 | chr11 | TATATGAAATTAAAAATAACTACACTGGACACTGG GCTCTGCATACAGTAAATATTCAGC[CG]TTGTCTG CAGACTTGCCTAAGTGTGTACGCAGTAGTTATTC TAGGCAGGTGCTGTGCTCC | 66 |
| cg01432450 | ACTN4 | chr19 | AGCAGGAGCTGCTAGATGCAGGCTGGGGCACAG GTGTTCCAGGCAGAGGGTGAAGCACTA[CG]AAG GCCCTGAGACAGGAAGAAGCTTGGCGTATTCAG GGACCAGCTAGAGAGGAGTGAGTG | 67 |
| cg06038342 | RASA3 | chr13 | GCCACAGGCAGCTGCTGAGGCCTGAGCCGGGG ACGAAGCTCCTCCCTGCATGGGACACG[CG]GG GCTGCAGCCACTCCCAGGCCTCCATCACACAGG GACGGTACAGAAATAGCGTGTCTTG | 68 |
| cg05903540 | TRAPPC9 | chr8 | GCTAACTGTCCATTTCCATGTCTGTTTTCATAGTG GACTTGGAGATCCACATCCTCACGG[CG]TGCCGC | 69 |
| | | | AGACCTGGACGCACCCTGCTCTCTCCTTTTCAGG CTCTGTGCCCAGCTCTTCCT | |
| cg23958370 | SORCS2 | chr4 | GGCCACGTTCGCAGGGGCAGGTATCAGGACACG GACATCTCTTCCTGGGGGCTGTGGTGC[CG]TCCA CTGCTGGGCCCCATTCCCTTCCTGCAGAGGGAG GGGCTGGCTCTGCTCTCTGTTTG | 70 |
| cg01624571 | SMAD3 | chr15 | CTCCAGTGTGTGTACGGAGAGCCTCTTTGAAGCG CTGTTGCCTTTCTGTGGCTAGATCCA[CG]ATCTTC TCTCTGGGATCACCCCATAGGACATTTGCTTTCC ACCTTCTCCAGTCCCAGTGA | 71 |
| cg15407373 | MBP | chr18 | GGAACGGCCTTGGCGAGGGAGCCCGGCCCTC CAGGCCACACCTACGGGGCTCCTAAGGC[CG]GG TTCTCGCCCACAGCAGAGACCCTGGCCACGCAT GGCCACCTGGCCTCCGGGATGCGTC | 72 |
| cg24426435 | URB1 | chr21 | CAAAGAACCGTGATATTTGCCCTCGTCTGCAAAT AGCACACACATTCTTTTATTAGAAA[CG]TATTTAA ATGTCAGGAGCCATTACTCATTGAATGGAATTTGT TGACACATGGATAAAGCA | 73 |
| cg17723057 | RP1-209A6.1 | chr6 | AGAAAGGGCCCAATCTACACAGAAGAAACTCTGT GGAAACAGACCCTACCACTCTACCAC[CG]TGCTG TACTTGATACACACCAAGAGTCTTACTGAGGTCA CACCCTTCACCAGGAATTTGA | 74 |
| cg07072176 | TRAPPC9 | chr8 | GATGTCACGTAGCACCACGTGATGCTCACAGAGC GGAACTCCTCCCTGCCCACAGGGGCA[CG]GGGC AGATGCAAGCCTGCTCCCAGATAACTCCGACCAG TCCTGTGAATTCTTCTGTGGCA | 75 |
| cg09411495 | PKD1 | chr16 | CTGGGCTTCTCAGCCTTATCCTGGGGATGTTCTG GGGCAGACAGTTGTCTGTCATGGGCC[CG]TCCT GTGCACTGCAAGACACGGACCTGTGTCCCTCCC CTCTGCCTACTGATGCCAGCAGC | 76 |
| cg14405768 | CLCN3P1 | chr9 | TACATAGGAATATCTCCTGTTTCACAACTTTGTTG TAAATTTTGACTCATATTTTCCCTC[CG]ATGCTGG GGATGTAAAAAAAAGTCAGCCTGACTATAAATAG AAGCACTGACAGTCTGACA | 77 |
| cg22234135 | UGT2B15 | chr4 | ATGCAAGACCACTTGCATCAGCAGCGTGTGTCAG CAAGATAGCAGAAGCAGGAAGAGAGC[CG]GCCA GAAGACACTTACCCTGGCTGGAAGAGATGTACCC CTGAAGATCAAGAAATAGGCCA | 78 |
| cg14758757 | SLC38A10 | chr17 | GGGGATGCGGGCCGGCATGTGCACCACTGCACC CACATGGCAGCTCCAGAGCGGAGCACA[CG]CAC CCTGGGGCCCGGCCACTCCACACAGGCACGCAC CCAGCAGAAACCACCCCCTGCCAA | 79 |
| cg18504656 | DACH1 | chr13 | GTTCGAATTTATCTGTGTGATTTATTTCCAATCCC AGCTGGTGGATTAGAGCCTGAGCAC[CG]CTCTCC TCCTTTTCCTCCCCACTAATCATCACTTCTAGTTA TTCTAGCAAATAAGTTACT | 80 |
| cg08459186 | SDK2 | chr17 | GTGACTGGGCGCTCAGTTAATACTCAGCCGCAGA TATTAATCCCTTTCTATGCAAAGAGA[CG]ATGGAT TGTGAGCCCTGAGAAATCAAAGTTGAAACTCAGC CAACCTCCTGCCCTGCTCGC | 81 |
| cg15619439 | WTAPP1 | chr11 | AATTGAGGAAGAACCAGCCCCAATGTGGTCATGC CATATGACACTGCAGAACCCCTAGTT[CG]GAATG | 82 |
| | | | GAGTCAAAACTGAAAAGATAAGGCAACCAGGCTG ATTTATTGTCTGGGTCCCTAG | |
| cg08599496 | SLC37A1 | chr21 | CAGAAAGAAGCTGCATCCCAGCCCAGATCCAGCT GAGAGCCACAGCTGCCAAGAGGCAGT[CG]GAGC TCTGGGCACCTCTCTGTGCACCCAGGAAGCCCC AGGAGGACTTCATCAAGCCAAGT | 83 |
| cg26379583 | MICAL3 | chr22 | CAAGTTTCTATCGGTAATCAATGTCTATGCCCATA TCATCTGCCTTCCCTCCAATGATAG[CG]AGCGAC ATCTGCCGCATGTCTCGCTGAAGCCAGCCTCTGC CTGTGCTTCTGATCCTGTCC | 84 |
| cg11694427 | FHOD1 | chr16 | CAAGCCCTGGCACAGCAGCCCAAATGGGGAGAA GGGGGAGGAGCCAGGCCCAGGAAGCAG[CG]CT CCTCATGCCTTGTCCGGCCTTGGTTGATTCTGGC AGGTGCTAACCTTGGCACAGCGGG | 85 |
| cg03071134 | NA | NA | TATGCAGCCAGGCATGGTCGCTCACACCTGTAAT CCCAACACTTTGGGAGGCCGAGGCTG[CG]GATC ACCTGAGGTCAGTTTGAGACCAACCTGACCAAGA AAGAGAAACTCCATCTCTACTA | 86 |
| cg03486689 | RP11-455G16.1 | chr4 | GTGCTCCAGTTCCCGGTGAGCCTCGGTACTGTG GCAGCAGTCAGTGTGTCTGGCGGGTGT[CG]GCG TGAAGCGGGGCTGGGCCAGCGGGAGGTAGCTCT GTGGGAGTGGAAGGCCTGTATTTC | 87 |
| cg22908413 | AP001619.2 | chr21 | CCTTCAATCTGCAAAATGGCAGGACTGTTCTTTC CTGGCCTTCTGTACAGATAATGACAT[CG]AAAATG ATATGAGAGCCACATCCTAAGTGAAAAGAGTTCT TTGTCAAATATAATTATTCT | 88 |
| cg04418936 | ESR1 | chr6 | TTCTTAGCAGGTAATAAATAAATCCCTGCCTGGAA ATTCTGCAGGAGAATTTCACCCCTG[CG]CTTTCA GGTTAAAAGATTGCATTCCCATGATGAAGATGTTC TGTGAATAGAATCATCTCA | 89 |
| cg23896850 | NA | NA | AGCTAGAGGATCCTGTGGGGAAAGTGCCGGGCA CACAGTAGGGGCTCACTCTTCTAGACA[CG]TTAT CTAAAACCTGGTTCATCTGTCCTTCCACGCAGGG CCTAGGGGATGCCGAATTCCAG | 90 |
| cg10075819 | LIMS1 | chr2 | ACTCCGGGGCTTGCGCTGCGGCTCCTGCGCCTG TCATGCACCCAGTTTCCCAGAAGCTTC[CG]GGGT TAGGTTGTCAGTGTTGAGAGGCAGTGCTAGTTCA GACTTGACCTTGTGATGGTCTG | 91 |
| cg18343899 | NFIA | chr1 | ACCACCCCCCTGTTAACGTCATTGTTCCCTTTCC CCCACCCCCACCGTCTTCCAGGAAGG[CG]GTTTG CTTGGCTCATCCCTTGGGTCTTGCATTGCAGTGA CCTTTGTAAGCCAAGAACTGC | 92 |
| cg21964649 | GPR133 | chr12 | TCTTACCAGCAGAGAAACTGAGCCCAGAGAGA GGGGGGCGCTTGCTGGTGGTCGCCCGT[CG]TTC AACAGCGTGTTATTGAATTACCGGTAGAGCCTGG GCTGTTTAGGTAGCACCCGGGCC | 93 |
| cg02136949 | TRAPPC9 | chr8 | GTCAAACAACTTGCTGGCTTTGAGTGACAGGCAG GATGCTAGCCCCAACCTGGGATGTCA[CG]TAGCA CCACGTGATGCTCACAGAGCGGAACTCCTCCCT GCCCACAGGGGCACGGGGCAGA | 94 |
| cg02004401 | AMZ1 | chr7 | TCAGCAAGGCCAAGTGCCTCGGCCAGGGCGGTG GGGTCAGCGCTGAGCGGGTCAAGAGCC[CG]CGC | 95 |
| | | | CGGGGGCCATCCCCCTGCGCTTTCTATCGAGTTA AACGGACCCCACAATATCTCCTT | |
| cg16976269 | MYO18B | chr22 | CTCCTGGTTACAGGTTCCAATTTCTCTGCAGCCA GCTGGCTTCACCACAGTAAGACAGTG[CG]TCTAG ACAGTTGGGAGGCTGCATTCCTCATTCTGAAACA TTTCCCCTTGATCTCCCTAGG | 96 |
| cg02285217 | SLC1A2 | chr11 | ATCTAGTTCTACTCATGCATGTGAAAGGTTCAGTT GCAATGACACCAGGTAGATGGCATA[CG]TGTCTA ATTTTTAAATCAGAAATCATGACATTTCAGCTGTG CCAAATAAGCTATCCCAGC | 97 |
| cg11586857 | LTA | chr6 | GCAGGTGAGGCTCTCCTGCCCCATCTCCTTGGG CTGCCCGTGCTTCGTGCTTTGGACTAC[CG]CCCA GCAGTGTCCTGCCCTCTGCCTGGGCCTCGGTCC CTCCTGCACCTGCTGCCTGGATC | 98 |
| cg17017564 | NEDD4L | chr18 | GAATACAGAACGGTAGGCAGATCTCTAAAGAGGC CATCAGGAATGTAAACAAACTTTGCT[CG]GTTTTT AAATGAAAGCCAGACCATAGTACTGGGATGCATT CAGTAGCAAATCTATGGTAA | 99 |
| cg19991458 | SMAD3 | chr15 | TGGTTGCTGATCCACCCGCTTCTTTCTTTATAGAG ATTAAGAGCGGCTCCAGTGTGTGTA[CG]GAGAGC CTCTTTGAAGCGCTGTTGCCTTTCTGTGGCTAGA TCCACGATCTTCTCTCTGGG | 100 |
| cg04971651 | SDK2 | chr17 | AAGGGGGAAAAAAATCAATCAACTGACCATTTTG TTCTCAGCCTGAATTTTGGCCCAGCA[CG]CATTC CAAGTGGCTGCAAATCCCAGTGACAGCATGCATG ACGCTTGGTATCTGGGTCACT | 101 |
| cg08219438 | NA | chr19 | CCTCCCGCCCCACCAAGCAATCATACCATTAGCC ACGGCTCAGCGGTGGCGGAAGGACC[CG]GCCT GACCCGGGCGGGGGCGGGGCACAGGCTGCGG CTCCCACCGAGACCCCTCTCTCCC | 102 |
| cg13703663 | AMZ1 | chr7 | TCTTGGGAAACCTAACGCTTTTCTGTTCCAGGTC CAACACAACGCCTCACAAAACGATCA[CG]AGATT GAAAGGCTGATGTCAGTGAACCATCCCCTTCCAG GGCACGCAGTGCCAGACCCTC | 103 |
| cg03472600 | MYOD1 | chr11 | TCTCAGGCCAGGTCCTGAAGTTTGACACCTTGGC GAAATGCACTTTCCTTGACTCAGCAC[CG]CGAGT GAGGCGGAGCCAAGCCCCGAGCAGAAGGGTTTT CTTCCCAGCTGAAGAGGCAGCT | 104 |
| cg27196158 | LINC01219 | chr11 | TGGGCCTGGGGAGTGAGGGCCCAGGCCGGGCT GGGTCTCGCTCCATAAACCCTGGCTGGC[CG]GA GCTGCAGACATCACTGTTGACACAGGGGTGATGT CACTGCCGGCCAATGGGGAGACCC | 105 |
| cg16144665 | POU6F1 | chr12 | TGAATGTGGGCTTCAGGCCCATGTCTACCGGCTG GATGGTTTCCAGGGCTAGGGGTCTAC[CG]GCTG GATGGTTTCCAGGGCTAGGGGTCTACCTGCTGG ATGGTTTCCAGGGCTAGGGGTCT | 106 |
| cg19450056 | NA | chr11 | CACCCGGCCCCAAGAGGCATCGGAGGCGCTG CTTGGCCCAGAGGAGAACAGCACTGATG[CG]GA GGCCCAGAGAGCTGGGGAGGCCTTGGGGGCGC CAATGGGAGGGTCTAAGAGCCCCCCG | 107 |
| cg22505202 | RP11-86H7.7 | chr1 | ACAGAAGGCGCAGGCCTGCGGAAGGGTGAGGC CAGTGGACCTGGGCAAGGCATTGGCAGA[CG]GT | 108 |
| | | | GTCATGCTCCCAGGCCTGGTGGAGCTGCCTGCA GCACAGAGACCAGCCCTCCTGAGGA | |
| cg26294431 | ERBB4 | chr2 | CGCACTTAATTTCTCATTTGGACCAAAACAAACTC CTCCAAACTGCTACTGACTGCTAAC[CG]CGAGAG GGGAGACGCGCAATATGTATTTAGTTGGGAACAC ACAGCCACACACATACATAG | 109 |
| cg12410029 | NA | chr9 | GGCATGAGCCACTGCGCCTGGCCAATTGCCAAT AAATTCCAATGTTTTAGGATCAGGCC[CG]CCATC CTCACCACGTTCCATCATAGCCTCATCTCATGCC TCCCTGTCTGGAGCCACTCTG | 110 |
| cg19260376 | PEAR1 | chr1 | CAGCCGGCCCCATGTGTCTCCCACCCCATCTCTC CATGTCCCTCAACAGTGTCTCTGCAT[CG]CTGTC TGCCCTAGAGCTCTTGTTCTGTCTTTGGGCTGTC TCTGTCTCTCTGTCACTGTGT | 111 |
| cg24411296 | ARSF | chrX | CCACCTTTCTGGACTAAGCCAATGTACATCTCAC ATGTGTCGATTGATGTCTCCTGTCCC[CG]TAAATT GTATAAAGCCAAGCTGTCACCCAACCACCTTGGG CACTTGTTCTCAGGACCTCC | 112 |
| cg16685895 | FAM134B | chr5 | AGCAAGGAGAGTGACAGGACCATTAGTCAGCAAA AGCAGATCACCTCACCAAGCAGGAAT[CG]CCAGC CTGGTTCACCCCTGGACCCTGACATGCCAACACG TCAACACACTCTAACTCCTCG | 113 |
| cg08876943 | NA | chr5 | GCTTCCTTTCACCTGGGCCTCCCCCTCCTCCCCT GAGCTTCCCTCACCGGCAGGCCAGCC[CG]GCCC TCAGGGACAGCTGGTGGGAAAGATGAGAATGAA CACGTCCAGCCCAGCCCTGAGCC | 114 |
| cg07177285 | PRKCA | chr17 | GGAAATGTGAGGCTGGAAAGCAACATAGGATTCT TCATTCCAGACAGCAGCCAGTGGGCC[CG]CTCAT GAAAAGTTGATGGTTGTGCTTAATGAGAGAAGAA AAAAGGCATCTAATAGCTAAA | 115 |
| cg22792232 | NA | chr3 | CCTACATTCACGATGACCCACTTCTCCAAAAATTT CACCATTGGATGGCCTCTCTTGTCA[CG]ACACCA AGGCCTCAGAGGTATGAAAAACACAGACCTCACC CAGAAGAAAAAGCTTATCCT | 116 |
| cg09134861 | CTB-49A3.2 | chr5 | ATGTTTGACTCCGTTCTAATAGTTATCTTTTCTTTC TAAACTTCTGTTCTTCCCACTTCA[CG]GCTCAACC TCTTCATGGGTTTGCTCTGTGATTTGAGAGAGCTT TTCTTGTTCTTTATCTT | 117 |
| cg10879755 | ZZEF1 | chr17 | GGGAAGCCAGGCGTCCCATCAGCCGGGAGACGA GGAGCTGTAAATCCAGCCCCAAGACA[CG]GCAA CATCGGGCAGCCCACAGGCAGTGACAGTGAATT TGTAGCCCCATTCGTTGTGACTG | 118 |
| cg20039512 | NA | chr6 | GGCCCTCAGTGCCCCTCAAGGCGAAAGGAGCGT GACGGATGAACCCTGAGTGGAAGGGGC[CG]CCT GTCCCAAACACGGACGGCTTTTCTGCAAGTTTT ACAAACTGATAAACCTTTGTGAA | 119 |
| cg01965462 | ERBB4 | chr2 | ATTCCTTCTGCTTAAGAAATTAGCATTTCAAAAAT ATGCCACTTTCCCCTGAACTCCAAG[CG]TGTCTA ATAATGCGAGAAGCAGATTGTCTTCCCTCGTTTC CTTAATTTGTTTCATCTAAA | 120 |
| cg18039042 | TSPAN9 | chr12 | CACAGTTCTAGGTGTGACATGAATACCTGCTGGG ATCCAGGGAGGACTTTTCACACCCAC[CG]CCCCA | 121 |
| | | | GGTAGGACATGAATTCTTCCATCCAGCCATCCCT ATTGAGCACCTCCCAGAGGCC | |
| cg01998808 | NA | chr16 | ACCCGGGTCTGTGGGGCTCCTGGCCTGCAGGC GCCGCCTCCTACTCCTGCTGGGGCCCC[CG]TCT CCTGGGCCTGCAGCAACCCAGGGCTGTGTTGTG TCTCCAGGCAACGGTGAGGTGTCC | 122 |
| cg08737246 | MBP | chr18 | ACACCCCGCGGGGCAGGGGATGGGGCAGGGGT CTCCGGGGACTTCCACAGCCTATGTCCA[CG]CG GCGCCTGGAGGGGAGCGGGAGGAGCAGGCAGG TTCCCACCCTCCGAGCCCAGGCAAGG | 123 |
| cg02165747 | NA | chr20 | AGAATGGGCCCCCTTTTTACTAAACAAGACGCCA AGTGGTCCTTGGAAATTTTCTCTTTA[CG]CTAATA ATGTAAGTGGGGACTCAAGTAATTGGAAAAAATA AAGGAGCAGTGACCTTAGTG | 124 |
| cg07900658 | CAGE1 | chr6 | AAAGTGGGGTACAGAAACGAAAACTCCGGGAAG AAACGGCCAGCACGGCCTCGCCGTGC[CG]GCT ACTCAACACGCCTTCCTGAGAGCACAGAACATCC ACAGCCCTATACAGCGCGCCATC | 125 |
| cg00591952 | KIF23 | chr15 | AGAGGGTGCCACTCTGGATGGGGAGAGGGTATA AAAGCCATTGTTGTGTGTTCGGAGGA[CG]TGGG AATACTGTTGGCAAACCATGAGTATGAATAAAGCT GCTTTGTGCATTTCTGCTTGT | 126 |
| cg19046661 | NCOR2 | chr12 | GAGCAACAGGAAATTTGCACAGTCTCAAAGAATC TCCCCGCAAGTATGTATGAATCATGG[CG]GTGGC TCCACAAATCTATATTCCTCCCTCCAGGAGGCGG AGCTTAACTCCCCTGCCCTGG | 127 |
| cg13659405 | NA | chr2 | GGAACTCCATGCCTTGATCTATGTTGTTCTTTGTG TCTTACCTTCCCTCACTGCTCAAGA[CG]CTGTCTT TAACCCTCAAGACACTGTCTTTCTTTAACCCTCAA GACTCATTAAGAAAGAGT | 128 |
| cg05728394 | OVOL2 | chr20 | ACAGGCAACTAATCTAGGAAATAAGATTGGTTTAC GTCTTCCTTTAATCCAGGATTCCAA[CG]GCCCTGT GTGCCTGAGGCAGAAGAGTTTGCCTAAAATATTT GTTAAGAGACTGGATGAGA | 129 |
| cg14619716 | RP11-494M8.4 | chr11 | GCTTGCAAGACTGCATTCTTAGGTGCTAGACTTC TCAATAGATAACTTCAAGGAGCCCAG[CG]CCAGG GAGTGAGGCCCTCAACAAACCTTTTGGTGGCAG GGCAGTGTGAGTTTGCCCACAT | 130 |
| cg22508597 | NA | chr2 | GGTCACACAGGGATTTAGTGTCAGAGCAGGATTT GGATCCACCCACCAAACCCAAGCAGA[CG]GCTC CTGTCCAGTGCCTGCACCCTGCCGTCTGTTCCTG TGAGAAGCCAGTCCGGGCCACC | 131 |
| cg06432980 | NA | chr9 | TCTTGTTTAGACAGTTCGGCAGTTAACTCTGTGTC TGTGTTTCTGAGCTCAGCCCTGCAC[CG]CATCTC CCTGCCCTCCCTTGCCAGCAGCTCAGATGAAAAA CACACTCATAAAGAAATCTT | 132 |
| cg02953381 | TNXB | chr6 | CACCCTGGGGCTCCCATCGTCCACTCACCTGTCA CCCCGATGGCAGACACGGGGCCCACA[CG]CTGG CCACCGTGGAAGCCGTACAGGTTCATCTTGTACT TGTTGTCTGGCTCCAGGCCGGA | 133 |
| cg04318178 | RAPGEF1 | chr9 | GGGGAGAGCAACAGAATAACCTAATCTTAAGAAT TCCTCTCTAATCCCCAGCTCAGAAAG[CG]TCTTTT | 134 |
| | | | ACAGTCATCCCCTCTGCAGTGTCCCAGACTAAGC AAACAAACAAAAAAACATAG | |
| cg14865309 | ING2 | chr4 | CTCAGGGGACCCGCCCGCCTCGGCCTCCGAAAG TGCTGGGATTACAGGCGTGAGCCACCG[CG]CCC AGCAAGCTTGCAGGTTTTTACATTTCTTTGAAGGC ACATGTTTCCTTTCACAAGAGT | 135 |
| cg19146067 | SRF | chr6 | TAGGGCTGTTGCTAGGGAGAGGGAAGAGGGAGA CCAAATGTCGGGGTTGGGGTGGAGGG[CG]TCA GGCAGAGGCAACTGACTTCATTTGTGCCACACGC ATGGGCATTGCAGCCTTGCGCTG | 136 |
| cg16935849 | RASA3 | chr13 | GGCAGGAAGTCAGCCCAGGAGGCTGTTGAGGAC CAGCAGGACCCAGTGGGATGCGCAGGG[CG]GG GCCTACACAGACGGGGGTTGGGGGGGGACTGG GAGCTCCACGACATGCCGGCCTCCTT | 137 |
| cg17813310 | AFF1 | chr4 | CAAACAAGGCTGGTAGACTTTTTATTTGAAAGGC AAGGATGGTGGGATGATACTAGCTAC[CG]AGAGG GAAATGACCTGGAAAACTAAGAACTCTAGTTGGG TCATGTATTAAGATCATGTGT | 138 |
| cg23353945 | RP4-541C22.5 | chr11 | CCTCAGTGATTTCATTTGACTTCGTTTTTAAATAA ACCTACCTGTCAGCAACTCCCCAA[CG]TGTACCT CCAGCCCAGGCTTTCTCCTGAACTCCAGGCTTAT ATGGGCCCCTGCTACACAG | 139 |
| cg02135693 | ARHGAP44 | chr17 | TCAAAAAAAAAAAAAAAAAAAAAGAAAAAAAGAC ATGATTGCTGCCCTTAAAAGTTCA[CG]TTCCAGT GAGCAAACAGGCCCTGACAAATAATGACAGCACT TGGTTATGCATACCCAGGG | 140 |
| cg11320084 | RNF2 | chr1 | GCAATTACACTTGCAGTTTTATCCTGCAAGGATGA AAAGTGTATGTTTGTTGTACGAGAG[CG]GAAGGG TGCCGAAGTTCATGTGAAGTTCTGCAAACCAAAG CCTGATGAGGATTTCCGCCG | 141 |
| cg10005146 | SSH2 | chr17 | TATAGCTCACTCCATTGTAAATATTATAAGCCCTC TGTGTCATGGGAATAACCCTGCCTA[CG]ACGTGA TGCTGATGTAATAATAATATCTAAAGCTAAGCACC TGGAGGAAGAAGAGAAAGA | 142 |
| cg08350488 | AIRN | chr6 | GAGCTGTCCAGGCGCGGAGCTGTCCAGGCGCG GGAGCTGTCCAGGCGCGGAGCTGTCCA[CG]AG GCACTGACCTTGGTTTCAGAGCCTCTGCAGGGCT TCTGTGTCCTTTTGGCTGAGCCGA | 143 |
| cg01204772 | NA | chr2 | TTCTCGAGGGAGGAACTGGACGCGTGCATGTT TGCATTCTCGAGGGAGGAACTGGACG[CG]TGC ATGTTTGCATTCTCGAGGGAGGACTGGGATGCAT TTATGTTTGCATTCTTGAGAGAG | 144 |
| cg20352178 | ANP32A | chr15 | GAACAGACAGGCTACTCCCACTGGCAGCCAGGC AAGAGGCTGGAGAACCACATCTGCCAT[CG]TCAC AGAGCTCTTCTATGTCCCCAGCTGCAGGAGAGTC ACACTGGGCTCTGTACACCGTC | 145 |
| cg22666034 | VPS45 | chr1 | GTTTCTAGGAATTATATATGGCATGAGTCATTGCT ATAGCATCACAGAAATTTTCATCAA[CG]TCTATCT AGAACAGTCAGGAGTTCTAGTTATGAGTTCTGTA ATAGATATTTATTGAACAT | 146 |
| cg14600450 | CUL9 | chr6 | TGACCAGCCAGCTTCCTCCATAGCATGACCTGTG CCAAATGTAACCATGGATTCTGCTGG[CG]CTGCC | 147 |
| | | | TCAAGTCCTGGAAGCCAAATCACAAAGACTATTA CAACTGCTCTGCCATGGTAAG | |
| cg16676436 | KCNAB1 | chr3 | TTAGAAACACACAACCAATTTTAGTTTTTCCAGCC CAGATTCCTTGATCAGATGCCAACC[CG]TCTCTC ACCAGATCCCTCCAGAAATATGCCCACACCAGAG CCCCACCCACCTCTTCCTAA | 148 |
| cg08574479 | NA | NA | GCGGGAGATACCAGAGACCCTAAACTGGCGTGA GGCCTGCTCGGCATGCACATGGGGTTA[CG]CGC ATGGTTTCTGGACTACATGTTCTGATTGGGTGAG AGAAAATCTCTAGGCCTTCTCTG | 149 |
| cg01274257 | NA | chr22 | GCCGTATTTATGAAGCTTTTTTCTCTCAACATCT ATTGAAAGAACCCACCGCCATGTTC[CG]GATTTC TAGGCCACACCTTGAAAACATGCAGAGCTCACAA TCCCTGCATCTGCCTCCTGC | 150 |
| cg13265003 | SLC37A1 | chr21 | GCTTAAGCAAACCACATGGTTAAGCAAACCCTGT TCTGGATCACATCATTAGCAGCTAGA[CG]TCTAG CTATAGCTATTAAATCCTAAGGTTTACAATTTGATT TCTATCCCATACGAATTAG | 151 |
| cg17178435 | AFF4 | chr5 | TGGTCTTGAACTCCTGACCCTGTAATCCCAGAGT GCTGGGATTACAGAAGTGAGCCACTA[CG]CCCG GCTGACTCACTTCTTTGTAACAGGAAGTCACTAA CACTAGATACTGACCATCAGCA | 152 |
| cg00817529 | SULT1A1 | chr16 | TTTTGTAGAGATGGGGTCTCACTGTGTTGCGCAG GCTGGTCTGAAACTGCTGAGCTCTAC[CG]ATCCT CTTGCCTCAGCCTCCCAAAATGCTGGGATTACAA GTGTGAGCCGGCTACTTCCCG | 153 |
| cg04708753 | CASP9 | chr1 | CGCCCTCAGGACGCACCTCTGCGCCTCGCCCCG CCCCCAGGGCCAAGCCTCCCATCACCG[CG]CCG CCCCAGAACACGCTCCGCGTCACCGCCCCGCCC CAGAACCCGCCCCACGTCACCGCC | 154 |
| cg08239103 | SOX6 | chr11 | AAAAAGCTATCTAGTTTTGCCACTATAACTGAAGG AATATGGTGGACAAGAGAAGAGAAA[CG]AGGTGT TGGTGATACATGAGAAAAGCAACAGAGGATGTCA CTATGCCTATGGAATTAATG | 155 |
| cg14316231 | KAT6A | chr8 | AAAAACCAACACTTAGCAGCTCTTCAGGTCAACT ACAGTTCACAATTCCCTGGAGGAAAA[CG]CAGCC CATAGTACAAGTCTGACAACACCTCTAGATCTGC GGAATCACCTATTCTACATAA | 156 |
| cg24322623 | MYOD1 | chr11 | TTGGCGCGAAGCTTTAGCAGCAATCTCCGATTCC TGTACAACCATAGCTGGGTTTCTAAG[CG]TCTAG GGAAGAAGGACTGGGCCCACGACCTGCTGAGCA ACTCCCAGGTCGGGGACTGGCG | 157 |
| cg15695155 | KDM2B | chr12 | CACTTGCCCCTTCCCACTAGAGCCCCAGGTGGC ACGGATGGGCAGCCTCCCCACCGCTCA[CG]CCC ATGCACCTTGGCACCTCCGGAGCCCGCAACACA TGCTTACCCCACTGACCTCTTTTC | 158 |
| cg20479209 | MROH5 | chr8 | TGATGATGTACTTCAAGTGGAAGGGGTCAGCACT GTTCATGTCCTGCAGCATCTTGAAGC[CG]GTGGC TAAGGTTGGCAAGAAGGCATGTGTAAGCAGGCT GTGGGTAGCTCACATTGTGGCA | 159 |
| cg02150218 | ARID1B | chr6 | GACACAACCATGCCATTCATTCTTCCCACCAAGC CAAAAGAAAATCTGGGTGTATCTGGA[CG]ACTCC | 160 |
| | | | TGTCTTCCCCTTCATATGCAGCCATTATGTTTCTA GAGCTAGAACCATCTGAAAA | |
| cg06182311 | NA | chr16 | TCAGGGCCCACCCTGCCTCGAGGTTGCTGTGGG ATACTGGGTGGATAACGGCGATACAGG[CG]CAAA CACGTGATGCTGTCGTGTTCTGCCATAAAAAGGC ATGAAGTCTGGACACGCGCTAC | 161 |
| cg15873830 | GALNT2 | chr1 | AGAGATTAAAAACAGCCCATTTATTTCTGTTTTC TCCCGCAGTAATATTTTTATGTCTC[CG]TTAACAA ATATGATGTTCGCCATTTAAATTATATGGAAGCAG CTTTGGATGATTTAGCAA | 162 |
| cg22553357 | FDFT1 | chr8 | CATGCCTGAGCCTCCCCCGCGCCGCCGCCGTGG GCTCCTGCGCGGCCCAAGCCTCCCCGA[CG]AGC GCCACCCCCTGTTCCACGGCGCCCAGTCCCGTC GACCGCCCAAGAGCTGAGGAGTGC | 163 |
| cg16899306 | HLA-DQB2 | chr6 | AAGATAGAGAGCATCTAAAAGCAGAATATGTTTAC CAGGTAAAAGAAACAGGGAAGAGA[CG]GTAGCA AGAGGTTTGCAATAGTGGCACATGAAAGCATTGA GCCACTCTAATATTCTGTAT | 164 |
| cg15400159 | FANCA | chr16 | CAGGAACGCAGTGACCATGCTGTCCAGCTGGCA GCTCTCGAATGCCTGGGCCATCAAACG[CG]CCAC CCAGTCTAGTTAAGAACCATGACATAGTCACAGC AAGGCAAGGGCAGCCAGCAGGA | 165 |
| cg24847366 | NCOR2 | chr12 | GATGGGGAAACCGAGGCCCAGAAAGAAAACAGC CCGCCTGAAGGTGACAGAGCGCCTCGC[CG]TAT CACCAGCAACCCCTCAAGATAACCTTATCACGTT TCACGGAGGGAGAGAGGGCGGCA | 166 |
| cg21616626 | DAG1 | chr3 | TAAGGAATTCAGTTTAGGGGGCAGTGGGACCA GGGAACCCATGATTAATAGTGTCTGCC[CG]CTAA GCGTGCTCACATAGTAGCCTGAGGAGAGAGGGA ATTCCTGAGCTGTGGTTTTCAGG | 167 |
| cg19597170 | NA | chr12 | CTTCTGGGAAGGAGGAGAGCAGACACAGAGACA CACAGGAGGGCGACACGGAGGCACAGG[CG]GA GACTGCAGTGGAATCATGCTTCCACAAGCCGAG GAACACCGACGGCTGCCGGCACACC | 168 |
| cg05763097 | EXOC3L4 | chr14 | GCTCCCGCCCCAGGTTTTCTAGGGGTCACCCATA CACAGTTCCAGAATTTGAAAAGTTTC[CG]CGGGG AGGTTCCCATGGGAGCAGGGCCTGTGCTGGTTC CACCAGGTGCCTTTGGGCGCAT | 169 |
| cg05809586 | KRTAP27-1 | chr21 | TTCTGATTGGCAAGGCTGAGAAACACAAGCCAGC CCTGCTGAAGAACTTTCTGATTGGCA[CG]CTGTC CTTTCGCAGGGCCTGGAATTGGAGTAAGTAGTTT GGACAACTCCGGGGAAGCAGT | 170 |
| cg26484001 | RP11-90B22.1 | chr10 | GTTACCTTTTGGCAGGCACATTTTCCTCCTGCTC GAATGCTATAAACACCCCAGCAGGGA[CG]ATAAA TAGCAATGTAGACAGCAAGGGAAAATATTTGCTA AAAGATACTCCTGCACTTTCT | 171 |
| cg03058016 | SLC35B4 | chr7 | ACTGAGTGATGATGTTCATGAGGAGGTAGAACCA CATGATGGGCAGGGTCACTCCGATGA[CG]GGAA TTTCATATAACTCTGTAGAGGTTACAAGAGGATAT AGCCATATTGCTTTTTCACAG | 172 |
| cg12598211 | PITPNM2 | chr12 | GCCTTCCCTCCAGAAGTGGGGGGTCTATCCTAG CAGCTCTGGTCACTCCCAGGTGGAGGC[CG]GGG | 173 |
| | | | AGAGAGCTGGTTGTGGACAAAGACAGATGTTCCA GTGAGGTCTCCTTTGAAGAATCC | |
| cg27191457 | ANP32A | chr15 | AGTGTTTTACATGTAGTGAGGGAGGAGCATGGTG TCGGTTTTTCACAAAACACAGGAATG[CG]TGTACA TACCAGGGAGTGGAGTAAAATGTATTTCTCACTA CTAGGAGCAGAAAGTTCGAA | 174 |
| cg09935045 | PPAP2B | chr1 | TTCGGCAAAGTGAAGAGTTGAGTACGCGCTCCG GACCGCTGTGCGGGGCTGCCTGACTTG[CG]GGA GGCCGAGGGGAGAGGCGGGAGCCCCGGCCTC CGGCGGGCGCGGGGCTCGGCGGCAG | 175 |
| cg00819310 | VANGL1 | chr1 | CTGGGAAAACTGGTTGCTGCATGGGTGGAGCATT TACAGTTCAGTTGCTTCACCCTCACT[CG]CTTGTT CCATGATAGAGACCCCAGAGAGGAGGTGTGGCT TTGTCATTTATCTTTCAGCCC | 176 |
| cg14531133 | Sep-12 | chr16 | CCCTCAACCACTCTGCTGGGGTGAGCAAACCCA GGCCCCACAGCTGGCTAGCGGATATC[CG]TGT CCACGATTGGCGCCATGTGTTTTAAGTTGCAGCG TCAAAAACATGAAGAGATTGCTT | 177 |
| cg18669346 | PXMP4 | chr20 | CGCAGCAGTGCGTTGACGACTACGAGCAGAGCC CTTAGCTGCGGCGGGGCTGCCATAGTG[CG]GGC TGCGCGCAGGGTCGGGGTTAGGAACTGTAAGCG CACTGACAGCCGGAGGTTCCAGCT | 178 |
| cg05046501 | C1QTNF3 | chr5 | GTATGACACTGAGTGAAATGCTTATTTATACAAAG ATAAATTTCTAAAATGTATGTTTAC[CG]TCAAAGA GCTAAAGACCTAGTGGAAGTTGATTGACAGAAGA GTTAAAGTTGCTTATGAAA | 179 |
| cg00694890 | CUL9 | chr6 | TCTGTGAGCAGCCCCTCCTTTTGTCCCTCCCAGT CTTGCTGGAATGAGTACCTGACAACT[CG]GATCG AGCAGAACCTTGTTTTGAATTGCACCTGCCCCAT TGCCGACTGCCCCGCCCAGCC | 180 |
| cg21340152 | FGGY | chr1 | TAAGGTGGCAGCCTGTCTTTTAAAAATTAATGGAT TCCAAATGCCATTCAAACCTATTTC[CG]CCCTTAA CCTCCTCCTCTTCTCCCCAACCCTGCCTGTTAGG AGTCTTATGTTCTGTATAA | 181 |
| cg07505941 | STK24 | chr13 | TTCGCCCCTGACTCCAGGTCTCACTGCGCTGACT CCAAGTGAGCTCACCCTTTGAGAACA[CG]CCCCA ACTTCCCAGGCCTTTTTGCTTTTGGAGGTTCCGC AAGTCAGGAAAATGCTTCACT | 182 |
| cg10794439 | AC113133. 1 | chr8 | GCATGAAGGGCCTCGGGGCAGCTCAGTACAGGA TGCCCCAGGGAGGATGGAGATCAGAGC[CG]GCC GCTGAAGCCCCCAGCACCACACACACCTGCTGG AGATGGTCGCACAGCGTCTTCATG | 183 |
| cg17829193 | AF015720.3 | chr21 | GTGAACTTTCTCTGACTCACCTGGGCTTCGGCCG GAAATAAGCAAAGTGCTGATACCTGA[CG]TAGCC GTGACCTGGGCAAGGTATTGTTTCACATGATCCT GTTCCTGAGTGCTTAGGCAAG | 184 |
| cg00332269 | CREB3L1 | chr11 | GCCTCTCAGTCTGGAAAATGGAGAGCAGAATATT CGCTATCCTCCTTAAAAATCCAGATA[CG]GGCCA TAAATCATGTGGAAATCATGTGTGCTCTGTTGCTC TGTGCAAAATGCAAAATGTA | 185 |
| cg14146112 | CDH4 | chr20 | GTTTCAGTGAATGAGCTGAGCTTTGCCAAAACCA GGCTTGGCAGGCCAAGGTCAAGGCTC[CG]TCCA | 186 |
| | | | GAAGAGGGCCCAGAGGAGACTGAGTGAAGTTCT TTCAAAAAGGGAGGCCACGTCAC | |
| cg05684145 | GAS2L1 | chr22 | GACCCGCAGCAGGAGCAGCAGCTGTTCCGGCGC CTGGAAGAGGAGTTCCTGGCCAATGCC[CG]GGC CCTTGAGGCTGTTGCTAGCGTGACCCCCACTGG ACCAGCCCCTGACCCAGCTCGGGC | 187 |
| cg09624870 | TACC2 | chr10 | CAGAGTGTGCAACAGCCAAATAGAACGCACGTCA AGTTTTGCAAACTGTCCAGCACAGCC[CG]CGCTG TTTCTGTGGTTAAGCGCCATTAATACCACACAGG TGAAGAAAAAAGGGCCGGAAT | 188 |
| cg06121748 | PARD3 | chr10 | ATCTGGTGGTGAAGGGAGAGTGTGGTTCTCACCT ATGTGAAGGTACCCCAGGTCTGTGAG[CG]TCCCC AAGGTCCAGAAAGCACTTGCCAGATGCTCAGAAC TGGATCAGCACTTTACTTAAT | 189 |
| cg08830374 | RP11-283G6.4 | chr12 | TATTTGAAAACGAAATTCCGGTAAATCTTTCGCAC CATCTGGCAGTGTGGTGATTGGCCG[CG]AGTTAG GAACAAGCCACTGAGCACACAAGAAAACAAAGCA GTGAAAACCCACCACCCCAA | 190 |
| cg14791502 | SDK1 | chr7 | GGCTTGTCAATGGCAGAGCCTTCCTGAAAGTCAG GTTCTCCTGTCAGGGTGGAGACCCAG[CG]GCTTT CAGGAGCAAAAAGAAGTCTCCAAACCGACACGC TTTGCTCAGCGTTGACATATA | 191 |
| cg01346767 | SCARF2 | chr22 | GCCGAGGGGGGAGCAGCCCTGCTGGGGGCCTA TAAATACATCTCCTCAGGCCACTAGAGT[CG]CCC CTGGAGGTCCCGCGGCGTGTGGGGCAGTGGGA GGCCATCAAAACCAGCTGACAGCTG | 192 |
| cg08790890 | NA | chr2 | TGGAGTGATTGGAATGAATGCCCCTCAGCCATTC AAAGGTTTACTAAACATATGAATCCA[CG]TGATAA AATGCTCGTCCTAGAAGCTTAAGTACAAAGAGCA GGCTACAAAATGGCCCGTGC | 193 |
| cg11874091 | LIMS1 | chr2 | AGTTTGCTGTTTGTATTGCTTCCAGTGCGTGTTGC TGTTCAACAGTACCCTTGGTTTCAG[CG]TAGTTGC TATGATGAAGAAAACCTGTTAGAATCTGCCTTTTT TTTTTTTCCAGAACTGTT | 194 |
| cg26312133 | SDK2 | chr17 | GAGGCAGCTGGCACCCGGGTTGGGGGAGCTGG CGGGGGGACAGCTCTGACAAGGTGATGA[CG]GG CATCCTGCGGCAGGATGCTTCTGGGGAGAGTCA CCTTCAAGAGAGTGAAGGGAAGTGA | 195 |
| cg08507070 | SLC4A4 | chr4 | TTTTGCTTCCTACAGGGAAGAGGCTGCTTTGAGA TGCTCTGCATTGTCTGTCAGAAAGAA[CG]AAGTTT CTAATTACTTGCTATGTATAGTCTTGGAGGGAATG TGAAATATGCATCAAAGAG | 196 |
| cg22229261 | NA | chr17 | TGCATCCTCCCCTTAATCCAAGTCAGGTAAATTG GGGAGAGAGGGCGTTAGAGAGTCAGA[CG]GAAG CCCCAGAGAGATGGTCAGTCACACCCTCAGATGT GACTAGAAATCCGGATGTCGGT | 197 |
| cg03111135 | PARD3 | chr10 | CACAATTAGTCTGCCAGTAATGCTTTTTCACTTTA ATGGTCTACACTAGGAACAGCACTG[CG]TGGTTT TCGTGAAAAGGTGCTCCATAAATGACAAGACTTTT TCCTTCTCTGAAGAAATAT | 198 |
| cg05886281 | NA | chr1 | CTCTGCACACACACACAAAGAGCTCGTGTGAGCA CACAGCAAGGTGGCAGCTACCTACAA[CG]CAGG | 199 |
| | | | AAGAAAGGGTTCACCAGAAACTATGCCGGCACCC TGATCTCAGACTTCCAGCCTCC | |
| cg20725021 | MASP1 | chr3 | ATCTTTGTTCTGAGGTCCCTGTGTGTCTCTGGAAT TGGCTGGCATTCTCCATTCAGGTCA[CG]CCTTCC CCTGCCTGCAACAGATCCCCTCTCCCCCTCTCTC CCTCTGGCTTTTCCTCTCTC | 200 |

**Table 4 -sets of predictive CpG sites:**

| **Set** | **No. of CpG sites** | **cgIDs (further information in Table 3)** |
|---|---|---|
| A | 18 | cg24688871, cg18379824, cg10094916, cg22165175, cg22277636, cg15834295, cg02754929, cg06223984, cg17972013, cg03980424, cg18888319, cg04438098, cg15637874, cg06757951, cg26881363, cg13957186, cg22175856, cg15224600 |
| B | 20 | cg06771789, cg20707157, cg16741230, cg16319140, cg26096304, cg00283857, cg08069338, cg22777490, cg09174638, cg00657415, cg18269134, cg01008088, cg17379497, cg06376130, cg05317498, cg03518863, cg20271602, cg03953789, cg20464719, cg22235661 |
| C | 20 | cg06771789, cg24688871, cg20707157, cg10094916, cg01312475, cg09218398, cg15985703, cg26096304, cg06509814, cg09174638, cg00657415, cg18269134, cg00492074, cg01008088, cg17379497, cg06376130, cg20271602, cg00100025, cg00698263, cg15214456 |
| D | 20 | cg06771789, cg24688871, cg20707157, cg01873983, cg16741230, cg01312475, cg08069338, cg08101922, cg22777490, cg09174638, cg00657415, cg10419493, cg00613587, cg06376130, cg20018782, cg10499189, cg03518863, cg20271602, cg00100025, cg01199135 |
| E | 20 | cg06771789, cg24688871, cg19712277, cg20707157, cg10094916, cg01312475, cg09218398, cg16319140, cg07671805, cg22810163, cg22777490, cg00657415, cg18269134, cg00492074, cg00613587, cg00100025, cg01199135, cg03953789, cg13668823, cg19571408 |
| F | 19 | cg06771789, cg19712277, cg20707157, cg10094916, cg09218398, cg15985703, cg00283857, cg15452389, cg02186126, cg22402224, cg08101922, cg18269134, cg00492074, cg06376130, cg05317498, cg03518863, cg20271602, cg03953789, cg19571408 |
| G | 20 | cg24688871, cg20707157, cg26096304, cg15562220, cg02186126, cg22402224, cg08101922, cg08211704, cg22777490, cg09174638, cg00657415, cg10419493, cg18269134, cg01008088, cg10499189, cg00100025, cg01199135, cg00698263, cg03953789, cg22235661 |
| H | 20 | cg24688871, cg10094916, cg01873983, cg16741230, cg16319140, cg18549952, cg08069338, cg15452389, cg08101922, cg00657415, cg18269134, cg00492074, cg06376130, cg03518863, cg20271602, cg00100025, cg01199135, cg03953789, cg20464719, cg19571408 |
| I | 20 | cg01873983, cg16741230, cg01312475, cg16319140, cg07671805, cg08069338, cg15452389, cg22810163, cg00657415, cg10419493, cg18269134, cg00492074, cg00613587, cg17379497, cg06376130, cg10499189, cg03518863, cg00100025, cg01199135, cg15214456 |
| J | 20 | cg06771789, cg24688871, cg19712277, cg16741230, cg01312475, cg09218398, cg16319140, cg18549952, cg26096304, cg15452389, cg08101922, cg18269134, cg17379497, cg06376130, cg10499189, cg00100025, cg00698263, cg03953789, cg13668823, cg19571408 |
| K | 20 | cg24688871, cg10094916, cg01873983, cg12642525, cg18549952, cg26096304, cg15562220, cg08069338, cg22402224, cg08101922, cg08211704, cg10419493, cg00492074, cg06376130, cg20018782, cg05317498, cg03953789, cg13668823, cg22235661, cg19571408 |
| L | 18 | cg06771789, cg19712277, cg10094916, cg01873983, cg16741230, cg01312475, cg09218398, cg08069338, cg22402224, cg22777490, cg10419493, cg00492074, cg01008088, cg10499189, cg00100025, cg01199135, cg22235661, cg15214456 |
| M | 18 | cg20707157, cg16741230, cg01312475, cg16319140, cg08069338, cg08101922, cg06509814, cg22777490, cg18269134, cg00492074, cg01008088, cg17379497, cg06376130, cg05317498, cg00100025, cg01199135, cg00698263, cg15214456 |
| N | 20 | cg24688871, cg01873983, cg12642525, cg16741230, cg16319140, cg15985703, cg00283857, cg08069338, cg15452389, cg08101922, cg06509814, cg18269134, cg00492074, cg01008088, cg06376130, cg20018782, cg03518863, cg01199135, cg00698263, cg13668823 |
| O | 20 | cg06771789, cg24688871, cg19712277, cg20707157, cg10094916, cg01873983, cg16741230, cg16319140, cg08069338, cg02186126, cg22402224, cg08211704, cg06509814, cg09174638, cg00492074, cg01008088, cg17379497, cg06376130, cg00698263, cg22235661 |
| P | 20 | cg06771789, cg01873983, cg16741230, cg01312475, cg09218398, cg08069338, cg02186126, cg08101922, cg22810163, cg08211704, cg22777490, cg00657415, cg18269134, cg00492074, cg00613587, cg03518863, cg20271602, cg00100025, cg01199135, cg19571408 |
| Q | 19 | cg20707157, cg01873983, cg16741230, cg09218398, cg16319140, cg26096304, cg08069338, cg08101922, cg22810163, cg22777490, cg18269134, cg01008088, cg06376130, cg10499189, cg03518863, cg20271602, cg00698263, cg15214456, cg19571408 |
| R | 20 | cg06771789, cg10094916, cg01873983, cg15985703, cg08069338, cg15452389, cg08211704, cg10419493, cg00492074, cg00613587, cg01008088, cg17379497, cg09680131, cg03518863, cg20271602, cg00100025, cg01199135, cg03953789, cg22235661, cg19571408 |

### Determining UV sensitivity by predicting MED

Once the RNA expression level of the genes (i.e. the at least 2, 5, 10, 18, or 20 genes selected from Table 1, Table 2, or set A, B, C, D, E, F, G, H, I, J, K, L, M, N, O, P, Q, or R in Table 2; or the 2 to 200, 2 to 150, 2 to 100, 2 to 50, 2 to 20, 5 to 200, to 150, 5 to 100, 5 to 50, 5 to 20, 10 to 200, 10 to 150, 10 to 100, 10 to 50, 10 to 20, 18 to 200, 18 to 150, 18 to 100, 18 to 50, 18 to 20, 20 to 200, 20 to 150, 20 to 100, or 20 to 50 genes selected from Table 1) has been determined, the method may further comprise a step of predicting the subject's MED based on the determined RNA expression levels using a machine learning model, wherein the machine learning model has been trained on data comprising known MEDs and corresponding known RNA expression levels of the same genes.

In certain embodiments, the methods of the present invention may comprise a step of training a machine learning model on data comprising known MEDs and corresponding known RNA expression levels of the same genes.

Once the methylation level of the CpG sites (i.e. the at least 2, 5, 10, 18, or 20 CpG sites selected from Table 3, Table 4, or set A, B, C, D, E, F, G, H, I, J, K, L, M, N, O, P, Q, or R in Table 4; or the 2 to 200, 2 to 150, 2 to 100, 2 to 50, 2 to 20, 5 to 200, to 150, 5 to 100, 5 to 50, 5 to 20, 10 to 200, 10 to 150, 10 to 100, 10 to 50, 10 to 20, 18 to 200, 18 to 150, 18 to 100, 18 to 50, 18 to 20, 20 to 200, 20 to 150, 20 to 100, or 20 to 50 CpG sites selected from Table 3) has been determined, the method may further comprise a step of predicting the subject's MED based on the determined methylation levels using a machine learning model, wherein the machine learning model has been trained on data comprising known MEDs and corresponding known methylation levels of the same CpG sites.

In certain embodiments, the methods of the present invention may comprise a step of training a machine learning model on data comprising known MEDs and corresponding known methylation levels of the same CpG sites.

As used herein, "corresponding" RNA expression/methylation levels and MEDs means RNA expression/ methylation levels and MEDs determined from the same subject. As used herein, "known" means previously determined. Hence, a "known MED and corresponding known RNA expression level" means an MED determined on a subject's skin and an RNA expression level determined using a skin sample from the same subject. In some embodiments, the known RNA expression levels, the known methylation levels, and/or the known MEDs are derived from at least 5, 10, 15, 20, 30, or 32 subjects.

Machine learning models may be used to determine predictive feature sets.

In certain embodiments, the machine learning model may be a supervised learning model, for example a support vector machine (SVM).

In certain embodiments, the machine learning model may perform sequential backward selection (SBS; also referred to as sequential feature elimination), sequential forward selection (SFS), exhaustive search, random search, or search using genetic algorithms.

In certain embodiments, the machine learning model may use a regression model, for example a Lasso regression model, a general linear model, a Lasso/ridge regression and elastic nets, decision trees, random forests, gradient boosting, or deep learning and neural networks.

As used herein, "absolute error" refers to the difference between a subject's MED determined by the known, standardised method and the subject's MED predicted using a method of the invention. As used herein, "MAE" ("mean absolute error") refers to the mean of a plurality of absolute errors.

In certain embodiments, the absolute error of a predicted MED may be about 25 mJ/cm² or less, about 20 mJ/cm² or less, about 15 mJ/cm² or less, about 10 mJ/cm² or less, or about 5 mJ/cm² or less.

### Personalised UV protection and therapy

Once the MED of a subject's skin has been predicted, this information may be used to provide recommendations that are personalised to the subject.

In certain embodiments, the methods of the invention may comprise a step of determining the maximum dose of UV radiation the subject can be exposed to before experiencing negative effects thereof. The negative effects may include skin damage (for example tanning, burning, premature aging) or DNA damage.

In certain embodiments, the methods of the invention may comprise a step of determining which UV protection substances or strategies are appropriate for the subject, for example sunscreens with particular SPFs or sunlight avoidance.

In certain embodiments, the methods of the invention may comprise a step of determining the minimum or optimal dose of UV radiation the subject should be exposed to in order to experience positive effects thereof. The positive effects may include vitamin D synthesis or treatment of a disease or condition. The disease or condition may be selected from the list consisting of: vitamin D deficiency; eczema; acne; psoriasis; graft-versus-host disease; vitiligo; mycosis fungoides; large-plaque parapsoriasis; and cutaneous T-cell lymphoma. The treatment may be in the presence of a psoralen (i.e. PUVA therapy).

In certain embodiments, the methods of the invention may comprise a step of administering an effective amount of a UV protectant to the subject. As used herein "an effective amount" means an amount effective to prevent or reduce damage to the subject's skin by UV radiation, including DNA damage.

In certain embodiments, a "UV protection substance" or "UV protectant" may be a chemical absorber (i.e. an organic chemical compound that absorbs UV light, for example salicalate, cinnimate, or benzophenone) or a physical blocker (i.e. inorganic particulates that reflect, scatter, or absorb UV light, for example Titanium Dioxide or Zinc Oxide).

Certain embodiments of the present invention may have one of more of the following effects:
- reduced risk of harm to the subject (no need for UV exposure) when determining UV sensitivity via MED;
- location-independent determination of UV sensitivity indicated by MED;
- increased objectivity in determining UV sensitivity indicated by MED;
- increased precision in determining UV sensitivity indicated by MED; and
- increased reproducibility in determining UV sensitivity indicated by MED (reduced bias from e.g. previous UV exposure).

For the avoidance of doubt, the present application is directed to subject-matter described in the following numbered paragraphs:
1. A method for predicting the minimal erythema dose (MED) of a subject's skin, the method comprising:
   i. determining the RNA expression levels of at least 2 genes selected from the genes in Table 1 in a skin sample obtained from the subject, and
   ii. predicting the subject's MED based on the determined RNA expression levels using a machine learning model trained on data comprising known MEDs and corresponding known RNA expression levels of the at least two genes.
2. A method for preventing damage to a subject's skin by UV radiation, the method comprising:
   i. receiving or obtaining a skin sample from a subject;
   ii. determining the RNA expression levels of at least 2 genes selected from the genes in Table 1 in a skin sample obtained from the subject;
   iii. predicting the subject's MED based on the determined RNA expression levels using a machine learning model trained on data comprising known MEDs and corresponding known RNA expression levels of the at least two genes; and
   iv. administering an effective amount of a UV protectant to the subject.
3. The method of paragraph 1 or 2, wherein the at least 2 genes comprise at least 5 genes.
4. The method of any preceding paragraph, wherein the at least 2 genes comprise at least 10 genes.
5. The method of any preceding paragraph, wherein the at least 2 genes comprise at least 18 genes.
6. The method of any preceding paragraph, wherein the at least 2 genes comprise at least 20 genes.
7. The method of any preceding paragraph, wherein the genes are selected from the genes in Table 2.
8. The method of any of paragraphs 1-7, wherein the genes are selected from the genes in set A, D, G, H, N, O, P, or R of Table 2.
9. The method of any of paragraphs 1-8, wherein the genes are selected from the genes in set A of Table 2.
10. The method of any of paragraphs 1-5, wherein the genes are selected from the genes in set B, C, E, F, I , J, K L, M, or Q in Table 2.
11. The method of any of paragraphs 1-7, wherein the genes comprise:
   i. ENSG00000197978 and ENSG00000277060;
   ii. ENSG00000197978 and ENSG00000100376;
   iii. ENSG00000197978 and ENSG00000172799;
   iv. ENSG00000197978 and ENSG00000166670; or
   v. ENSG00000172799 and ENSG00000159247.
12. The method of any of paragraphs 1-7, wherein the genes comprise:
   i. ENSG00000197978, ENSG00000277060, ENSG00000100376, ENSG00000166670, and ENSG00000172799;
   ii. ENSG00000197978, ENSG00000159247, ENSG00000100376, ENSG00000166670, and ENSG00000172799;
   iii. ENSG00000277060, ENSG00000100376, ENSG00000166670, ENSG00000172799, and ENSG00000159247;
   iv. ENSG00000197978, ENSG00000100376, ENSG00000166670, ENSG00000106392, and ENSG00000159247; or
   v. ENSG00000197978, ENSG00000100376, ENSG00000172799, ENSG00000159247, and ENSG00000260075.
13. The method of any of paragraphs 1-7, wherein the genes comprise:
   i. ENSG00000197978, ENSG00000277060, ENSG00000100376, ENSG00000166670, ENSG00000172799, ENSG00000106392, ENSG00000112139, ENSG00000130779, ENSG00000159247, and ENSG00000260075;
   ii. ENSG00000197978, ENSG00000277060, ENSG00000166670, ENSG00000172799, ENSG00000106392, ENSG00000159247, ENSG00000100376, ENSG00000260075, ENSG00000188818, and ENSG00000169282;
   iii. ENSG00000166670, ENSG00000172799, ENSG00000106392, ENSG00000100376, ENSG00000260075, ENSG00000188818, ENSG00000115602, ENSG00000112139, ENSG00000224472, and ENSG00000233913; or
   iv. ENSG00000277060, ENSG00000166670, ENSG00000106392, ENSG00000159247, ENSG00000100376, ENSG00000260075, ENSG00000169282, ENSG00000126890, ENSG00000176933, and ENSG00000134184.
14. The method of any of paragraphs 1-7, wherein the genes comprise the genes in set A, B, C, D, E, F, G, H, I , J, K L, M, N, O, P, Q, or R in Table 2.
15. The method of any of paragraphs 1-7, wherein the genes comprise the genes in set A in Table 2.
16. The method of any preceding paragraph, wherein the genes comprise up to 200,150, 100, 50, or 20 genes.
17. The method of any preceding paragraph, wherein the sample comprises, consists, or consists essentially of epidermis cells and/or dermis cells.
18. The method of any preceding paragraph, wherein the subject is phototype I-IV on the Fitzpatrick scale.
19. The method of any preceding paragraph, wherein the machine learning model is a support vector machine (SVM).
20. The method of any preceding paragraph, wherein the machine learning model performs sequential backward selection (SBS).
21. The method of any preceding paragraph, wherein the absolute error is about 25 mJ/cm² or less, about 20 mJ/cm² or less, about 15 mJ/cm² or less, about 10 mJ/cm² or less, or about 5 mJ/cm² or less.
22. The method of any preceding paragraph, further comprising:
   i. determining the maximum dose of UV radiation the subject can be exposed to before experiencing negative effects thereof;
   ii. determining an appropriate UV protection substance or strategy for the subject;
   iii. determining the minimum dose of UV radiation the subject should be exposed to in order to experience positive effects thereof; and/or
   iv. determining the appropriate UV dose for treating a disease or condition susceptible to UV therapy in the subject.
23. A computer program comprising instructions which, when the program is executed by a computer, cause the computer to carry out the following steps:
   i. inputting the RNA expression levels of at least two genes selected from the genes in Table 1 in a skin sample obtained from the subject;
   ii. predicting the subject's MED based on the determined RNA expression levels using a machine learning model trained on data comprising known MEDs and corresponding known RNA expression levels of the at least two genes; and
   iii. outputting the predicted MED.
24. The computer program of paragraph 22, wherein the at least 2 genes are at least 5 genes.
25. The computer program of any of paragraphs 22-23, wherein the at least 2 genes are at least 10 genes.
26. The computer program of any of paragraphs 22-24, wherein the at least 2 genes are at least 18 genes.
27. The computer program of any of paragraphs 22-25, wherein the at least 2 genes are at least 20 genes.
28. The computer program of any of paragraphs 22-26, wherein the genes are selected from the genes in Table 2.
29. The computer program of any of paragraphs 22-27, wherein the genes are selected from the genes in set A, D, G, H, N, O, P, or R of Table 2.
30. The computer program of any of paragraphs 22-28, wherein the genes are selected from the genes in set A of Table 2.
31. The computer program of any of paragraphs 22-25, wherein the genes are selected from the genes in set B, C, E, F, I , J, K L, M, or Q in Table 2.
32. The computer program of any of paragraphs 22-27, wherein the genes comprise:
   i. ENSG00000197978 and ENSG00000277060;
   ii. ENSG00000197978 and ENSG00000100376;
   iii. ENSG00000197978 and ENSG00000172799;
   iv. ENSG00000197978 and ENSG00000166670; or
   v. ENSG00000172799 and ENSG00000159247.
33. The computer program of any of paragraphs 22-27, wherein the genes comprise:
   i. ENSG00000197978, ENSG00000277060, ENSG00000100376, ENSG00000166670, and ENSG00000172799;
   ii. ENSG00000197978, ENSG00000159247, ENSG00000100376, ENSG00000166670, and ENSG00000172799;
   iii. ENSG00000277060, ENSG00000100376, ENSG00000166670, ENSG00000172799, and ENSG00000159247;
   iv. ENSG00000197978, ENSG00000100376, ENSG00000166670, ENSG00000106392, and ENSG00000159247; or
   v. ENSG00000197978, ENSG00000100376, ENSG00000172799, ENSG00000159247, and ENSG00000260075.
34. The computer program of any of paragraphs 22-27, wherein the genes comprise:
   i. ENSG00000197978, ENSG00000277060, ENSG00000100376, ENSG00000166670, ENSG00000172799, ENSG00000106392, ENSG00000112139, ENSG00000130779, ENSG00000159247, and ENSG00000260075;
   ii. ENSG00000197978, ENSG00000277060, ENSG00000166670, ENSG00000172799, ENSG00000106392, ENSG00000159247, ENSG00000100376, ENSG00000260075, ENSG00000188818, and ENSG00000169282;
   iii. ENSG00000166670, ENSG00000172799, ENSG00000106392, ENSG00000100376, ENSG00000260075, ENSG00000188818, ENSG00000115602, ENSG00000112139, ENSG00000224472, and ENSG00000233913; or
   iv. ENSG00000277060, ENSG00000166670, ENSG00000106392, ENSG00000159247, ENSG00000100376, ENSG00000260075, ENSG00000169282, ENSG00000126890, ENSG00000176933, and ENSG00000134184.
35. The computer program of any of paragraphs 22-27, wherein the genes comprise the genes in set A, B, C, D, E, F, G, H, I , J, K L, M, N, O, P, Q, or R in Table 2.
36. The computer program of any of paragraphs 22-27, wherein the genes comprise the genes in set A in Table 2.
37. The computer program of any of paragraphs 22-35, wherein the genes comprise up to 200,150, 100, 50, or 20 genes.
38. The computer program of any of paragraphs 22-36, wherein the sample comprised, consisted of, or consisted essentially of epidermis cells and/or dermis cells.
39. The computer program of any of paragraphs 22-37, wherein the subject is phototype I-IV on the Fitzpatrick scale.
40. The computer program of any of paragraphs 22-38, wherein the machine learning model is a support vector machine (SVM).
41. The computer program of any of paragraphs 22-39, wherein the machine learning model performs sequential backward selection (SBS).
42. The computer program of any of paragraphs 22-40, wherein the absolute error is about 25 mJ/cm² or less, about 20 mJ/cm² or less, about 15 mJ/cm² or less, about 10 mJ/cm² or less, or about 5 mJ/cm² or less.
43. A computer-readable medium comprising the computer program of any of paragraphs 22-41.
44. A method for predicting the minimal erythema dose (MED) of a subject's skin, the method comprising:
   i. determining the methylation levels of at least 2 CpG sites selected from the CpG sites in Table 3 in a skin sample obtained from the subject, and
   ii. predicting the subject's MED based on the determined methylation levels using a machine learning model trained on data comprising known MEDs and corresponding known methylation levels of the at least two CpG sites.
45. A method for preventing damage to a subject's skin by UV radiation, the method comprising:
   i. receiving or obtaining a skin sample from a subject;
   ii. determining the methylation levels of at least 2 CpG sites selected from the CpG sites in Table 3 in a skin sample obtained from the subject;
   iii. predicting the subject's MED based on the determined methylation levels using a machine learning model trained on data comprising known MEDs and corresponding known methylation levels of the at least two CpG sites; and
   iv. administering an effective amount of a UV protectant to the subject.
46. The method of paragraph 44 or 45, wherein the at least 2 CpG sites comprise at least 5 CpG sites.
47. The method of any preceding paragraph, wherein the at least 2 CpG sites comprise at least 10 CpG sites.
48. The method of any preceding paragraph, wherein the at least 2 CpG sites comprise at least 18 CpG sites.
49. The method of any preceding paragraph, wherein the at least 2 CpG sites comprise at least 20 CpG sites.
50. The method of any preceding paragraph, wherein the CpG sites are selected from the CpG sites in Table 4.
51. The method of any of paragraphs 44-50, wherein the CpG sites are selected from the CpG sites in set B, C, D, E, G, H, I, J, K, N, O, P, or R in Table 4.
52. The method of any of paragraphs 44-48, wherein the CpG sites are selected from the CpG sites in set A, F, L, M, or Q in Table 4.
53. The method of any of paragraphs 44-48, wherein the CpG sites are selected from the CpG sites in set A of Table 4.
54. The method of any of paragraphs 44-50, wherein the CpG sites comprise:
   i. cg06376130 and cg03953789;
   ii. cg18269134 and cg00613587;
   iii. cg06376130 and cg01199135;
   iv. cg03953789 and cg00492074; or
   v. cg06376130 cg18269134.
55. The method of any of paragraphs 44-50, wherein the CpG sites comprise:
   i. cg06376130, cg03953789, cg18269134, cg00613587, and cg01199135;
   ii. cg06376130, cg18269134, cg00613587, cg01199135, and cg00492074;
   iii. cg03953789, cg00613587, cg01199135, cg00492074, and cg20271602;
   iv. cg03953789, cg18269134, cg01199135, cg20707157, and cg22235661; or
   v. cg06376130, cg03953789, cg01199135, cg00492074, and cg10094916.
56. The method of any of paragraphs 44-50, wherein the CpG sites comprise:
   i. cg06376130, cg03953789, cg00613587, cg20707157, cg09218398, cg26096304, cg09174638, cg00492074, cg20271602, and cg22235661;
   ii. cg03953789, cg18269134, cg00613587, cg01199135, cg20707157, cg00492074, cg20271602, cg22235661, cg24688871, and cg10094916;
   iii. cg06376130, cg03953789, cg01199135, cg20707157, cg09218398, cg00492074, cg20271602, cg22235661, cg17972013, and cg15224600; or
   iv. cg18269134, cg00613587, cg01199135, cg20707157, cg00492074, cg20271602, cg22235661, cg24688871, and cg10094916.
57. The method of any of paragraphs 44-50, wherein the CpG sites comprise the CpG sites in set A, B, C, D, E, F, G, H, I , J, K L, M, N, O, P, Q, or R in Table 4.
58. The method of any of paragraphs 44-50, wherein the CpG sites comprise the CpG sites in set A in Table 4.
59. The method of any preceding paragraph, wherein the CpG sites comprise up to 200, 150, 100, 50, 20, or 18 CpG sites.
60. The method of any preceding paragraph, wherein the sample comprises, consists, or consists essentially of epidermis cells and/or dermis cells.
61. The method of any preceding paragraph, wherein the subject is phototype I-IV on the Fitzpatrick scale.
62. The method of any preceding paragraph, wherein the machine learning model is a support vector machine (SVM).
63. The method of any preceding paragraph, wherein the machine learning model performs sequential backward selection (SBS).
64. The method of any preceding paragraph, wherein the absolute error is about 25 mJ/cm² or less, about 20 mJ/cm² or less, about 15 mJ/cm² or less, about 10 mJ/cm² or less, or about 5 mJ/cm² or less.
65. The method of any preceding paragraph, further comprising:
   i. determining the maximum dose of UV radiation the subject can be exposed to before experiencing negative effects thereof;
   ii. determining an appropriate UV protection substance or strategy for the subject;
   iii. determining the minimum dose of UV radiation the subject should be exposed to in order to experience positive effects thereof; and/or
   iv. determining the appropriate UV dose for treating a disease or condition susceptible to UV therapy in the subject.
66. A computer program comprising instructions which, when the program is executed by a computer, cause the computer to carry out the following steps:
   i. inputting the methylation levels of at least two CpG sites selected from the CpG sites in Table 3 in a skin sample obtained from the subject;
   ii. predicting the subject's MED based on the determined methylation levels using a machine learning model trained on data comprising known MEDs and corresponding known methylation levels of the at least two CpG sites; and
   iii. outputting the predicted MED.
67. The computer program of paragraph 66, wherein the at least 2 CpG sites are at least 5 CpG sites.
68. The computer program of any of paragraphs 66-67, wherein the at least 2 CpG sites are at least 10 CpG sites.
69. The computer program of any of paragraphs 66-68, wherein the at least 2 CpG sites are at least 18 CpG sites.
70. The computer program of any of paragraphs 66-69, wherein the at least 2 CpG sites are at least 20 CpG sites.
71. The computer program of any of paragraphs 66-70, wherein the CpG sites are selected from the CpG sites in Table 4.
72. The computer program of any of paragraphs 66-71, wherein the CpG sites are selected from the CpG sites in set B, C, D, E, G, H, I, J, K, N, O, P, or R of Table 4.
73. The computer program of any of paragraphs 66-69, wherein the CpG sites are selected from the CpG sites in set A, F, L, M, or Q in Table 4.
74. The computer program of any of paragraphs 66-69, wherein the CpG sites are selected from the CpG sites in set A of Table 4.
75. The computer program of any of paragraphs 66-71, wherein the CpG sites comprise:
   i. cg06376130 and cg03953789;
   ii. cg18269134 and cg00613587;
   iii. cg06376130 and cg01199135;
   iv. cg03953789 and cg00492074; or
   v. cg06376130 cg18269134.
76. The computer program of any of paragraphs 66-71, wherein the CpG sites comprise:
   i. cg06376130, cg03953789, cg18269134, cg00613587, and cg01199135;
   ii. cg06376130, cg18269134, cg00613587, cg01199135, and cg00492074;
   iii. cg03953789, cg00613587, cg01199135, cg00492074, and cg20271602;
   iv. cg03953789, cg18269134, cg01199135, cg20707157, and cg22235661; or
   v. cg06376130, cg03953789, cg01199135, cg00492074, and cg10094916.
77. The computer program of any of paragraphs 66-71, wherein the CpG sites comprise:
   i. cg06376130, cg03953789, cg00613587, cg20707157, cg09218398, cg26096304, cg09174638, cg00492074, cg20271602, and cg22235661;
   ii. cg03953789, cg18269134, cg00613587, cg01199135, cg20707157, cg00492074, cg20271602, cg22235661, cg24688871, and cg10094916;
   iii. cg06376130, cg03953789, cg01199135, cg20707157, cg09218398, cg00492074, cg20271602, cg22235661, cg17972013, and cg15224600; or
   iv. cg18269134, cg00613587, cg01199135, cg20707157, cg00492074, cg20271602, cg22235661, cg24688871, and cg10094916.
78. The computer program of any of paragraphs 66-71, wherein the CpG sites comprise the CpG sites in set A, B, C, D, E, F, G, H, I , J, K L, M, N, O, P, Q, or R in Table 4.
79. The computer program of any of paragraphs 66-71, wherein the CpG sites comprise the CpG sites in set A in Table 4.
80. The computer program of any of paragraphs 66-79, wherein the CpG sites comprise up to 200,150, 100, 50, 20, or 18 CpG sites.
81. The computer program of any of paragraphs 66-80, wherein the sample comprised, consisted of, or consisted essentially of epidermis cells and/or dermis cells.
82. The computer program of any of paragraphs 66-81, wherein the subject is phototype I-IV on the Fitzpatrick scale.
83. The computer program of any of paragraphs 66-82, wherein the machine learning model is a support vector machine (SVM).
84. The computer program of any of paragraphs 66-83, wherein the machine learning model performs sequential backward selection (SBS).
85. The computer program of any of paragraphs 66-84, wherein the absolute error is about 25 mJ/cm² or less, about 20 mJ/cm² or less, about 15 mJ/cm² or less, about 10 mJ/cm² or less, or about 5 mJ/cm² or less.
86. A computer-readable medium comprising the computer program of any of paragraphs 66-85.

It should be noted that the present invention may comprise any combination of features and/to limitations referred to herein, except for combinations of such features which are mutually exclusive. The foregoing description is directed to particular embodiments of the present invention for the purpose of illustrating it. It will be apparent, however, to one skilled in the art, that many modifications and variations to the embodiments described herein are possible. All such modifications and variations are intended to be within the scope of the present invention, as defined in the appended claims.

### EXAMPLES

### Methods

### MED determination using the known, standardised method

MED was determined as previously described (Heckman et al (2013) Minimal Erythema Dose (MED) Testing, J Vis Exp. (75): 50175) and outlined below. This method has been standardised as International Standard ISO 2444:2010.

The study sites were located on the subjects' lower backs since this area is rarely exposed to sunlight. The sites were split into control and test areas.

The first irradiation of the test sites was performed using a SOL 500 full spectrum solar simulator (Hönle UV Technology). Intensities were chosen individually to reach 0.9 MED for all subjects (i.e. 90% of the MED in a given test subject).

Irradiation to 0.9 MED was repeated twice more in the same manner, each time 24 hours apart, so that all test sites had been irradiated three times.

### Skin sampling

24 hours after the final irradiation session of each subject, four suction blisters of 7 mm diameter were taken from both test sites (irradiated) and control sites (not irradiated), as previously described (Südel et al. (2003) Tight control of matrix metalloproteinase-1 activity in human skin. Photochem Photobiol. 78: 355-60).

By taking samples from both test and control sites, the inventors enabled the identification of markers that allow reliable determination of UV sensitivity via MED prediction, regardless of recent UV exposure.

A single sample of the epidermis from each test site was sufficient to perform transcriptome sequencing and methylation profiling, as described below.

### Nucleic acid extraction

Tissue samples were suspended in the respective lysis buffers for RNA or DNA extraction and homogenized using an MM 301 bead mill (Retsch®). DNA was then extracted using the QIAamp® DNA Investigator Kit (Qiagen®) according to manufacturer's instructions. RNA was extracted using the RNeasy® Fibrous Tissue Mini Kit (Qiagen®) according to manufacturer's instructions. RNA and DNA samples were used for transcriptome sequencing and methylation profiling, respectively, as described below.

### Transcriptome sequencing

Transcriptome libraries were prepared using TruSeq Library Prep Kit (Illumina®) and sequencing performed at 1x50 bp on Illumina's® HiSeq system to a final sequencing depth of 100 million reads per sample.

Sequencing data was processed using a custom pipeline including Fastqc v0.11.767 for quality control, Trimmomatic v0.3668 for trimming and Salmon v0.8.169 for read mapping and quantification.

This method determined the level of 38,892 transcripts.

### Methylation profiling

Methylation profiling was performed using Illumina's® Infinium Methylation EPIC arrays (Love et al. (2014) Moderated estimation of fold change and dispersion for RNA-seq data with DESeq2, Genome Biol. 15:550).

Methylation data was processed using the minfi package (Aryee (2014) Minfi: a flexible and comprehensive Bioconductor package for the analysis of Infinium DNA methylation microarrays, Bioinformatics 30:1363-1369) in R. Normalization was carried out using the funnorm normalization method.

This method determined the methylation level of over 866,091 human CpG sites throughout the genome with single nucleotide resolution.

### Identifying features correlating with MED

Data from test (irradiated) and control (not irradiated) samples were pooled before analysis.

The 200 most strongly MED-correlated features (genes or CpG sites) of each dataset (from a total of 38,892 genes and 866,091 CpG sites) were selected via absolute Pearson correlation coefficient.

### Machine learning models for predicting MED

Sequential backwards selection (also referred to as sequential feature elimination) was used in a support vector model (SVM) to reduce the sets of 200 predictive features to sets of fewer features, for example 20 features.

Lasso regression models (Tibshirani (1994) Regression Shrinkage and Selection Via the Lasso, J R Stat Soc Ser B. 58:267-288) for the prediction of MED were built in R using the machine learning framework mlr (Bischl et al. (2016) mlr: Machine Learning in R, J Mach Learn Res. 17:1-5). First, models were built using the 200 most strongly MED-correlated features during the learning process. These were then cut down to produce a model with fewer features, for example 20 features.

The models were then used to predict MED based on subjects' RNA expression levels.

In examples, the parameters of the support vector models were as follows:
- Kernel: Radial-basis function
- Gamma: 0.05
- Cost: 1
- Epsilon: 0.1

Parameters to be set for SBS are the maximum number of features to retain and a threshold parameter determining the minimal value of improvement needed for a feature to be eliminated from the model. The inventors' models did not use the first parameter, but the second parameter was set to 0.01.

Model predictions and accuracy scores were extracted from leave-one-out-cross-validation (LOOCV) to avoid overfitting.

The feature selection and training included data from both test and control (irradiated and non-irradiated) samples, in order to allow accurate MED predictions irrespective of previous UV exposure of the sample.

### EXAMPLE 1 - MED determination using known methods

This study recruited 32 healthy female subjects belonging to Fitzpatrick phototypes I to IV (12 subjects belonging to phototype I and II, 10 to phototype III; and 10 to phototype IV). The subjects were aged between 30 and 65 years, with homogeneous age distributions in each phototype group.

Possible recruits were excluded is they had tattoos or scars in the area of skin to be exposed to UV radiation, if they had pigmentation disorders, if they were pregnant, or if they had taken anti-histamine or anti-inflammatory medication within two weeks prior to study start.

The MED of each of the 32 subjects was determined using the known, standardised method described above.

MED values ranged from approximately 50 mJ/cm² to approximately 210 mJ/cm². As expected from previously published data, stratification of donors using the Fitzpatrick classification system was an inaccurate predictor of MED. For example, the measured MED values for the subjects of phototype IV varied from 99.7 to 210.4 mJ/cm².

### EXAMPLE 2 - MED prediction using RNA expression levels

### Example 2a - 200 predictive genes

Skin samples were taken from the 32 subjects and the RNA extracted. The transcriptomes of each subject were then sequenced and the data processed, as described above.

From these data, the inventors identified the 200 genes whose RNA expression levels each exhibited a strong linear correlation with the MED determined by the known, standardised method (used in Example 1). These 200 genes are provided in Table 1.

### Example 2b - predicting MED using 200 genes

A machine learning model was trained on data comprising (i) the MEDs and (ii) the corresponding RNA expression levels of the 200 genes in Table 1, from all 32 subjects. This model was then used to predict MED based on RNA expression levels of the 200 genes in Table 1 in skin samples obtained from subjects.

Using the set of 200 genes in Table 1, MAEs of 16-18 mJ/cm² were consistently achieved.

### Example 2c - predicting MED using 150, 100, or 50 genes

The inventors then tested how accurately MED could be predicted using models trained on smaller sets of genes selected from the 200 genes in Table 1.

When sets of 150 genes selected from Table 1 were used, MAEs of approximately 13-15 mJ/cm² were achieved.

When sets of 100 genes selected from Table 1 were used, MAEs of approximately 11-13 mJ/cm² were achieved.

When sets of 50 genes selected from Table 1 were used, MAEs of approximately 9-11 mJ/cm² were achieved.

### Example 2d - predicting MED using 18-20 genes

High accuracies (low MAEs) were achieved by using sets of 18-20 genes selected from Table 1.

When sets of 18-20 genes selected from Table 1 were used, MAEs of approximately 8-13 mJ/cm² were achieved.

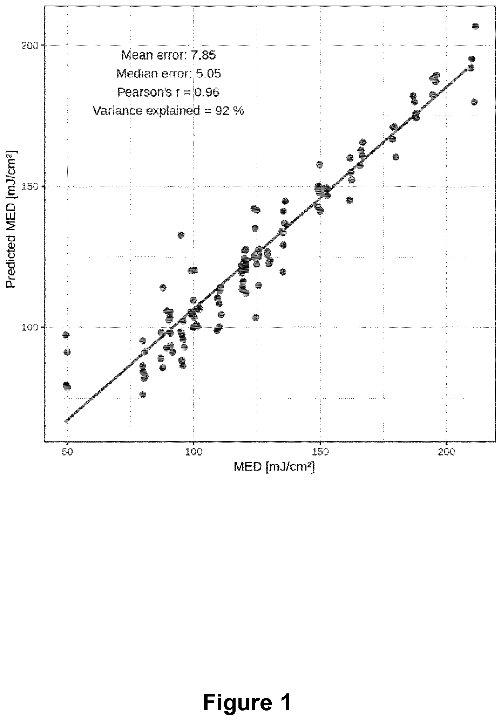

For example, the set of 20 genes in set A in Table 2 achieved a very low MAE of 7.85 mJ/cm² (see Figure 1).

### Example 2e - predicting MED using 10 genes

When sets of 10 genes selected from Table 1 were used, MAEs of approximately 10-15 mJ/cm² were achieved.

For example, the set of 10 genes ENSG00000197978, ENSG00000277060, ENSG00000100376, ENSG00000166670, ENSG00000172799, ENSG00000106392, ENSG00000112139, ENSG00000130779, ENSG00000159247, and ENSG00000260075 achieved an MAE of 9.71 mJ/cm².

### Example 2f - predicting MED using 5 genes

When sets of 5 genes selected from Table 1 were used, MAEs of approximately 13-20 mJ/cm² were achieved.

For example, the set of 5 genes ENSG00000197978 (GOLGA6L9), ENSG00000277060 (NLRP2), ENSG00000100376 (FAM118A), ENSG00000166670 (MMP10), and ENSG00000172799, which achieved an MAE of 12.76 mJ/cm².

### Example 2g - predicting MED using 2 genes

Surprisingly and advantageously, as few as 2 genes selected from Table 1 could be used to accurately predict MAE. When sets of 2 genes were used, MAEs of approximately 20-25 mJ/cm² were achieved.

For example, the set of 2 genes ENSG00000197978 and ENSG00000277060 achieved an MAE of 19.62 mJ/cm2.

### EXAMPLE 3 - MED prediction using methylation levels

### Example 3a - 200 predictive CpG sites

Skin samples were taken from the 32 subjects and the DNA extracted. DNa methylation profiling was performed and the data processed, as described above.

From these data, the inventors identified the 200 CpG sites whose methylation levels each exhibited a strong linear correlation with the MED determined by the known, standardised method (used in Example 1). These 200 CpG sites are provided in Table 3.

### Example 3b - predicting MED using 200 CpG sites

A machine learning model was trained on data comprising (i) the MEDs and (ii) the corresponding methylation levels of the 200 CpG sites in Table 3, from all 32 subjects. This model was then used to predict MED based on methylation levels of the 200 CpG sites in Table 3 in skin samples obtained from subjects.

Using the 200 CpG sites in Table 3, MAEs of approximately 9-11 mJ/cm² were consistently achieved.

### Example 3c - predicting MED using 150, 100, or 50 CpG sites

The inventors then tested how accurately MED could be predicted using models trained on smaller sets of CpG sites selected from the 200 CpG sites in Table 3.

When sets of 150 CpG sites selected from Table 3 were used, MAEs of approximately 7-8 mJ/cm² were achieved.

When sets of 100 CpG sites selected from Table 3 were used, MAEs of approximately 6-7 mJ/cm² were achieved.

When sets of 50 CpG sites selected from Table 3 were used, MAEs of approximately 4-6 mJ/cm² were achieved.

### Example 3d - predicting MED using 18-20 CpG sites

High accuracies (low MAEs) were achieved by using sets of 18-20 CpGs selected from Table 1.

When sets of 18-20 CpGs were used, MAEs of approximately 4-7 mJ/cm² were achieved.

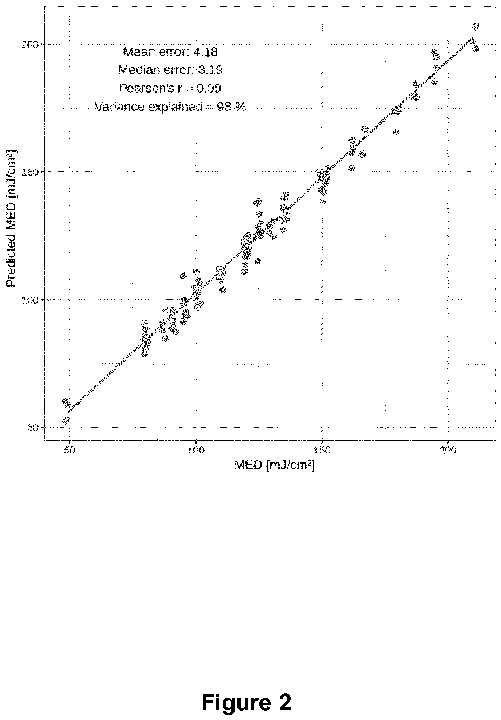

For example, the set of 18 CpG sites in set A in Table 4 achieved a very low MAE of only 4.18 mJ/cm² (see Figure 2).

### Example 3e - predicting MED using 10 CpG sites

When sets of 10 CpG sites selected from Table 3 were used, MAEs of approximately 7-12 mJ/cm² were achieved.

For example, the set of 10 CpG sites cg06376130, cg03953789, cg00613587, cg20707157, cg09218398, cg26096304, cg09174638, cg00492074, cg20271602, and cg22235661 (described further in Table 3) achieved an MAE of 6.81 mJ/cm².

### Example 3f - predicting MED using 5 CpG sites

When sets of 5 CpG sires selected from Table 3 were used, MAEs of approximately 11-15 mJ/cm² were achieved.

For example, the set of 5 CpG sites cg06376130, cg03953789, cg18269134, cg00613587, and cg01199135 (described further in Table 3) achieved an MAE of 11.01 mJ/cm².

### Example 3f - predicting MED using 2 CpG sites

Surprisingly and advantageously, as few as 2 CpG sites selected from Table 3 could be used to accurately predict MAE. When sets of 2 CpG sites were used, MAEs of approximately 15-21 mJ/cm² were achieved.

For example, the set of 2 CpG sites cg06376130 and cg03953789 (described further in Table 3) achieved an MAE of 15.58 mJ/cm².

## Claims

1. A method for predicting the minimal erythema dose (MED) of a subject's skin, the method comprising:
a)
i) determining the methylation levels of at least 2 CpG sites selected from the genes in Table 3 in a skin sample obtained from the subject, and
ii) predicting the subject's MED based on the determined methylation levels using a machine learning model trained on data comprising known MEDs and corresponding known methylation levels of the at least 2 CpG sites;
and/or
b)
i) determining the RNA expression levels of at least 2 genes selected from the genes in Table 1 in a skin sample obtained from the subject, and
ii) predicting the subject's MED based on the determined RNA expression levels using a machine learning model trained on data comprising known MEDs and corresponding known RNA expression levels of the at least 2 genes.

2. The method of claim 1, wherein:
a) the at least 2 CpG sites comprise at least 5, 10, or 18 CpG sites; and/or
b) the at least 2 genes comprise at least 5, 10, or 18 genes.

3. The method claim 1 or 2, wherein:
a) the CpG sites are selected from the CpG sites in Table 4; and/or
b) the genes are selected from the genes in Table 2.

4. The method claim 3, wherein:
a) the CpG sites are selected from the CpG sites in set A, B, C, D, E, F, G, H, I, J, K, L, M, N, O, P, Q, or R in Table 4; and/or
b) the genes are selected from the genes in set A, B, C, D, E, F, G, H, I, J, K, L, M, N, O, P, Q, or R in Table 2.

5. The method of claim 4, wherein:
a) the CpG sites are selected from the CpG sites in set A in Table 4; and/or
b) the genes are selected from the genes in set A in Table 2.

6. The method of any of claims 1-5, wherein :
a) the CpG sites comprise:
i) cg06376130 and cg03953789;
ii) cg18269134 and cg00613587;
iii) cg06376130 and cg01199135;
iv) cg03953789 and cg00492074; or
v) cg06376130 cg18269134;
and/or
b) the genes comprise:
i) ENSG00000197978 and ENSG00000277060;
ii) ENSG00000197978 and ENSG00000100376;
iii) ENSG00000197978 and ENSG00000172799;
iv) ENSG00000197978 and ENSG00000166670; or
v) ENSG00000172799 and ENSG00000159247.

7. The method of any of claims 1-5, wherein:
a) the CpG sites comprise:
i) cg06376130, cg03953789, cg18269134, cg00613587, and cg01199135;
ii) cg06376130, cg18269134, cg00613587, cg01199135, and cg00492074;
iii) cg03953789, cg00613587, cg01199135, cg00492074, and cg20271602;
iv) cg03953789, cg18269134, cg01199135, cg20707157, and cg22235661; or
v) cg06376130, cg03953789, cg01199135, cg00492074, and cg10094916;
and/or
b) the genes comprise:
i) ENSG00000197978, ENSG00000277060, ENSG00000100376, ENSG00000166670, and ENSG00000172799;
ii) ENSG00000197978, ENSG00000159247, ENSG00000100376, ENSG00000166670, and ENSG00000172799;
iii) ENSG00000277060, ENSG00000100376, ENSG00000166670, ENSG00000172799, and ENSG00000159247;
iv) ENSG00000197978, ENSG00000100376, ENSG00000166670, ENSG00000106392, and ENSG00000159247; or
v) ENSG00000197978, ENSG00000100376, ENSG00000172799, ENSG00000159247, and ENSG00000260075.

8. The method of any of claims 1-5, wherein:
a) the CpG sites comprise:
i) cg06376130, cg03953789, cg00613587, cg20707157, cg09218398, cg26096304, cg09174638, cg00492074, cg20271602, and cg22235661;
ii) cg03953789, cg18269134, cg00613587, cg01199135, cg20707157, cg00492074, cg20271602, cg22235661, cg24688871, and cg10094916;
iii) cg06376130, cg03953789, cg01199135, cg20707157, cg09218398, cg00492074, cg20271602, cg22235661, cg17972013, and cg15224600; or
iv) cg18269134, cg00613587, cg01199135, cg20707157, cg00492074, cg20271602, cg22235661, cg24688871, and cg10094916;
and/or
b) the genes comprise:
i) ENSG00000197978, ENSG00000277060, ENSG00000100376, ENSG00000166670, ENSG00000172799, ENSG00000106392, ENSG00000112139, ENSG00000130779, ENSG00000159247, and ENSG00000260075;
ii) ENSG00000197978, ENSG00000277060, ENSG00000166670, ENSG00000172799, ENSG00000106392, ENSG00000159247, ENSG00000100376, ENSG00000260075, ENSG00000188818, and ENSG00000169282;
iii) ENSG00000166670, ENSG00000172799, ENSG00000106392, ENSG00000100376, ENSG00000260075, ENSG00000188818, ENSG00000115602, ENSG00000112139, ENSG00000224472, and ENSG00000233913; or
iv) ENSG00000277060, ENSG00000166670, ENSG00000106392, ENSG00000159247, ENSG00000100376, ENSG00000260075, ENSG00000169282, ENSG00000126890, ENSG00000176933, and ENSG00000134184.

9. The method of any of claims 1-5, wherein:
a) the CpG sites comprise the CpG sites provided in set A, B, C, D, E, F, G, H, I, J, K, L, M, N, O, P, Q, or R in Table 4; and/or
b) the genes comprise the genes provided in set A, B, C, D, E, F, G, H, I, J, K, L, M, N, O, P, Q, or R in Table 2.

10. The method of any of claims 1-5, wherein:
a) the CpG sites comprise the CpG sites provided in set A in Table 4; and/or
b) the genes comprise the genes provided in set A in Table 2.

11. The method of any preceding claim, wherein:
a) the CpG sites comprise up to 200, 100, 50, 20, or 18 CpG sites; and/or
b) the genes comprise up to 200, 100, 50, or 20 genes.

12. The method of any preceding claim, wherein the sample comprises, consists, or consists essentially of epidermis cells and/or dermis cells.

13. The method of any preceding claim, wherein the subject is phototype I-IV on the Fitzpatrick scale.

14. The method of any preceding claim, wherein the machine learning model is a support vector machine (SVM).

15. The method of any preceding claim, wherein the machine learning model performs sequential backward selection (SBS).

16. The method of any preceding claim, wherein the absolute error is about 25 mJ/cm² or less, about 20 mJ/cm² or less, about 15 mJ/cm² or less, about 10 mJ/cm² or less, or about 5 mJ/cm² or less.

17. The method of any preceding claim, further comprising:
a) determining the maximum dose of UV radiation the subject can be exposed to before experiencing negative effects thereof;
b) determining an appropriate UV protection substance or strategy for the subject;
c) determining the minimum dose of UV radiation the subject should be exposed to in order to experience positive effects thereof; and/or
d) determining the appropriate UV dose for treating a disease or condition susceptible to UV therapy in the subject.

18. A computer program comprising instructions which, when the program is executed by a computer, cause the computer to carry out the following steps:
a)
i) inputting the methylation levels of at least two CpG sites selected from the genes in Table 3 in a skin sample obtained from the subject,
ii) predicting the subject's MED based on the determined methylation levels using a machine learning model trained on data comprising known MEDs and corresponding known methylation levels of the at least two CpG sites, and
iii) outputting the predicted MED;
and/or
b)
i) inputting the RNA expression levels of at least two genes selected from the genes in Table 1 in a skin sample obtained from the subject,
ii) predicting the subject's MED based on the determined RNA expression levels using a machine learning model trained on data comprising known MEDs and corresponding known RNA expression levels of the at least two genes, and
iii) outputting the predicted MED.
